# EUROPEAN PATENT APPLICATION

(11) **EP 4 455 117 A1**
(43) Date of publication of application: **30.10.2024**
(21) Application number: 22911348.5
(22) Date of filing: 22.12.2022
(51) Int. Cl.: C07C 33/38, C07C 33/50, C07C 69/96, C07D 207/46, C07F 7/18

(54) **AMINE-PROTECTING GROUP**

(30) Priority: 24.12.2021 JP 2021211613
(71) Applicant: SynCrest Inc., Fujisawa-shi, Kanagawa 251-8555 (JP)
(72) Inventor: XU, Pengyu, Fujisawa-shi, Kanagawa 251-8555 (JP); YONEYAMA, Shin, Fujisawa-shi, Kanagawa 251-8555 (JP)
(74) Representative: Meissner Bolte Partnerschaft mbB
(86) International application number: PCT/JP2022/047380
(87) International publication number: WO 2023/120646

(57) **Abstract**

An object of the present invention is to provide novel amine-protecting groups useful for the synthesis of special amino acids. Provided is an amine-protecting group.

## Description

### Technical Field

The present invention relates to amine-protecting groups.

### Background Art

NPL 1 discloses that a coloring reagent is used for the detection of free terminal amino groups in the solid-phase synthesis of peptides, and that the free amino groups are protected with tert-butoxycarbonyl protecting groups (BOC).

### Citation List

### Non-patent Literature

NPL 1: Anal. Biochem., 34, 595 (1970)

### Summary of Invention

### Technical Problem

An object of the present invention is to provide novel amine-protecting groups useful for the synthesis of special amino acids.

### Solution to Problem

As a result of extensive studies to achieve the above object, the present inventors succeeded in producing novel amine-protecting groups useful for the synthesis of special amino acids by introducing triple bonds into 9-fluorenylmethyloxycarbonyl protecting groups (Fmoc).

Upon further studies based on this finding, the present inventors have completed the present invention.

Specifically, the present invention includes amine-protecting groups described below.

### Item 1.

A compound represented by the following formula (1): 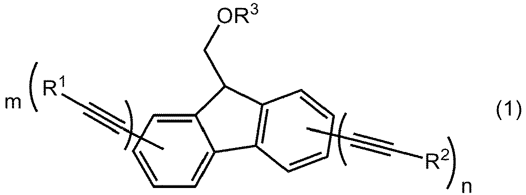 wherein
in formula (1), R¹ and R² are the same or different and are each a hydrogen atom, a halogen atom, an alkyl group, a trimethylsilyl group, an acetyl group, a benzoyl group, an ester group, an alkyl ester group, an aryl group, an alkyl alcohol group, a nitrile group, a group represented by 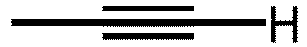 or a group represented by 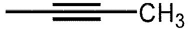
in formula (1), R³ is a hydrogen atom, a halogenated carbonyl group, or an active ester group;
in formula (1), m is an integer of 1 to 4; and
in formula (1), n is an integer of 0 to 4.

### Item 2.

The compound according to Item 1, represented by the following formula (2): 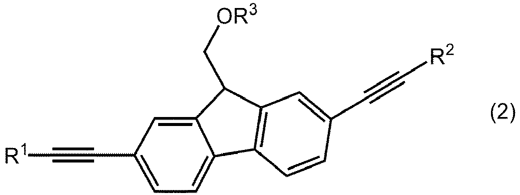 wherein
in formula (2), R¹ and R² are the same or different and are each a hydrogen atom, a halogen atom, an alkyl group, a trimethylsilyl group, an acetyl group, a benzoyl group, an ester group, an alkyl ester group, an aryl group, an alkyl alcohol group, a nitrile group, a group represented by 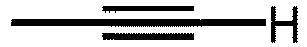 or a group represented by 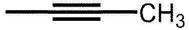
in formula (2), R³ is a hydrogen atom, a halogenated carbonyl group, or an active ester group.

### Item 3.

The compound according to Item 1 or 2, wherein the active ester group is an N-hydroxysuccinimide ester group, a pentafluorophenyl ester group, a 4-nitrophenyl ester group, a 2,4-dinitrophenyl ester group, an N-hydroxyphthalimide ester group, or a 1-hydroxybenzotriazole ester group.

### Item 4.

The compound according to Item 1 or 2, wherein the active ester group is an N-hydroxysuccinimide ester group or a pentafluorophenyl ester group.

### Item 5.

A compound represented by the following formula (3): 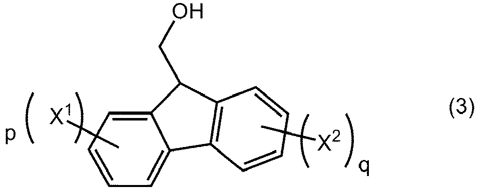 wherein
in formula (3), X¹ and X² are the same or different and are each a halogen atom;
in formula (3), p is an integer of 1 to 4; and
in formula (3), q is an integer of 0 to 4.

### Item 6.

The compound according to Item 5, wherein in formula (3), X¹ and X² are each an iodine atom.

The compounds represented by formulas (1) and (2) are excellent as amine-protecting groups in controlling the ease of deprotection.

The compounds represented by formulas (1) and (2) are excellent as amine-protecting groups in controlling pKa at the α-position of 9-fluorenylmethyloxycarbonyl protecting groups (Fmoc) and improving the yield of peptides.

The compounds represented by formulas (1) and (2) as amine-protecting groups are excellent in tracking various reactions based thereon because the triple bonds introduced can be detected by Raman spectroscopy. In particular, the compound represented by formula (2) (compound with alkyne groups bonded to positions 2 and 7) is particularly excellent in tracking various reactions based thereon because the triple bonds introduced can be highly detected by Raman spectroscopy.

### Advantageous Effects of Invention

The present invention can provide novel amine-protecting groups useful for the synthesis of special amino acids.

### Description of Embodiments

The present invention is described in detail below.

In the present specification, the terms "comprise" and "contain" include the concepts of "comprise," "contain," "consist essentially of," and "consist of."

In the present specification, a numerical range indicated by "A to B" means "A or more and B or less."

In the present specification, indications such as "part" and "%" are generally used.

In the present specification, values are expressed in parts by mass or mass% (wt%) unless otherwise specified.

### [1] Amine-Protecting Group

The amine-protecting group of the present invention is a compound represented by the following formula (1).

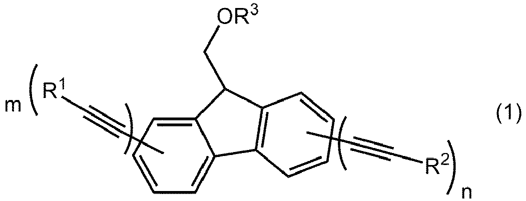

In formula (1), R¹ and R² are the same or different and are each a hydrogen atom (H), a halogen atom, an alkyl group, a trimethylsilyl group (TMS, -Si(CH₃)₃), an acetyl group (Ac), a benzoyl group (Bz), an ester group, an alkyl ester group, an aryl group, alkyl alcohol group, a nitrile group (CN), a group represented by 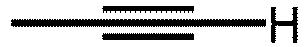 or a group represented by

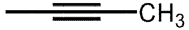

The halogen atom is preferably a fluorine atom (F), a chlorine atom (Cl), a bromine atom (Br), an iodine atom (I), or the like.

The alkyl group is preferably a C₁₋₈ linear or branched alkyl group, such as a methyl group (-CH₃, Me), an ethyl group (Et), an n-butyl group (n-Bu), or a t-butyl group (t-Bu); a C₃₋₁₂ cyclic alkyl group, such as a cyclopropyl group, a cyclobutyl group, a cyclopentyl group, a cyclohexyl group, a cycloheptyl group, or a cyclooctyl group; or the like.

The ester group is preferably a methyl ester group (COOMe), an ethyl ester group (COOEt), or the like.

The alkyl ester group is preferably a 3-carboxypropyl group ((CH₂)₃COOH), a 3-methyloxycarbonylpropyl group ((CH₂)₃COOMe), or the like.

The aryl group is preferably a phenyl group (Ph), a 4-methyloxycarbonylbenzene group (4-COOMe-C₆H₄), a 4-formylbenzene group (4-CHO-C₆H₄), a 4-methylhydroxybenzene group (4-CH₂OH-C₄H₆), a 3,5-dimethoxybenzene group (3,5-OMe-C₆H₄), or the like.

The alkyl alcohols (CH₂OH, (CH₂)₂OH, and (CH₂)₃OH), CH₂OCO₂(4-NO₂-C₄H₆), and (CH₂)₃OCO₂(4-NO₂-C₄H₆) are preferably a methylhydroxy group (CH₂OH), an ethylhydroxy group ((CH₂)₂OH), a propylhydroxy group ((CH₂)₃OH), a methyl(4-nitrobenzo)oxycarbonyl group (CH₂OCO₂(4-NO₂-C₄H₆)), a propyl (4-nitrobenzo) oxycarbonyl group ((CH₂)₃OCO₂(4-NO₂-C₄H₆)), and the like.

In formula (1), R³ is a hydrogen atom, a halogenated carbonyl group (-C(=O)-halogen), or an active ester group.

The active ester group refers to a highly reactive ester group, and is preferably an N-hydroxysuccinimide ester group (-C(=O)-O-Su), a pentafluorophenyl ester group (-C(=O)-O-C₆F₅), a 4-nitrophenyl ester group (-C(=O)-O-C₆H₄-NO₂), a 2,4-dinitrophenyl ester group (-C(=O)-O-C₆H₃-2NO₂), an N-hydroxyphthalimide ester group (-C(=O)-O-PTI), a 1-hydroxybenzotriazole ester group (-C(=O)-O-BTA), or the like.

The active ester group is more preferably an N-hydroxysuccinimide ester group or a pentafluorophenyl ester group, and particularly preferably an N-hydroxysuccinimide ester group.

| |
|---|
| Halogenated carbonyl group |
| 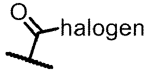 |

| | | |
|---|---|---|
| N-hydroxysuccinimide ester group | Pentafluorophenyl ester group | 4-Nitrophenyl ester group |
| 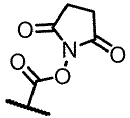 | 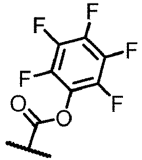 | 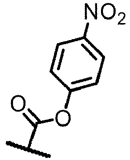 |

| | | |
|---|---|---|
| 2,4-Dinitrophenyl ester group | N-hydroxyphthalimid e ester group | 1-Hydroxybenzotriazole ester group |
| 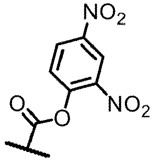 | 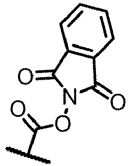 | 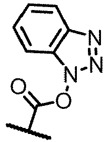 |

The halogenated carbonyl group (-C(=O)-halogen atom) is preferably carbonyl fluoride (-COF), carbonyl chloride (-COCl), carbonyl bromide (-COBr), carbonyl iodide (-COI), or the like.

The amine-protecting group of the present invention is an activated compound when R³ in formula (1) is a halogenated carbonyl group or an active ester group.

In formula (1), m is an integer of 1 to 4.

In formula (1), n is an integer of 0 to 4.

The amine-protecting group of the present invention is preferably a compound represented by the following formula (2).

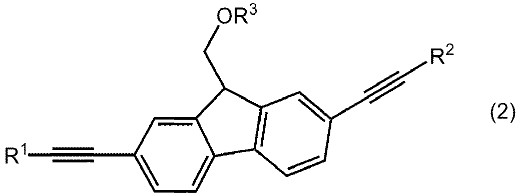

In formula (2), R¹ and R² are the same or different and are each a hydrogen atom, a halogen atom, an alkyl group, a trimethylsilyl group, an acetyl group, a benzoyl group, an ester group, an alkyl ester group, an aryl group, an alkyl alcohol group, a nitrile group, a group represented by 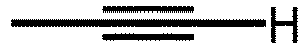 or a group represented by

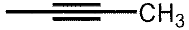

In formula (2), R³ is a hydrogen atom, a halogenated carbonyl group, or an active ester group.

In formula (2), R¹, R², and R³ are the same as those in the compound represented by formula (1).

In the amine-protecting groups of the present invention (the compounds represented by formulas (1) and (2)), triple bonds are introduced into their 9-fluorenylmethyloxycarbonyl protecting groups (Fmoc). The triple bonds introduced into the amine-protecting groups are conjugated with Fmoc to change pKa at the α-position of Fmoc. Due to this feature, the amine-protecting groups of the present invention can control the ease of removal of protecting groups from target amino acids.

The pKa at the α-position of Fmoc of the amine-protecting group changes to form, for example, an amine-protecting group that can be removed even by a weak base. The pKa at the α-position of Fmoc of the amine-protecting group changes to form, for example, an amine-protecting group that can be removed even under strongly acidic conditions. It is possible to remove all protecting groups in one step in the deprotection of peptides.

The triple bonds introduced into the amine-protecting groups of the present invention develop quantifiable peaks in the silent region in Raman spectroscopy. Due to this feature, the amine-protecting groups of the present invention can be quantified without being disturbed by other impurities.

Of the amine-protecting groups of the present invention, particularly the compound represented by formula (2) (compound with alkyne groups bonded to positions 2 and 7) is particularly excellent in tracking various reactions as an amine-protecting group because the triple bonds introduced can be highly detected by Raman spectroscopy.

### [2] Amine-Protecting Group Intermediate

The present invention includes an amine-protecting group intermediate represented by the following formula (3).

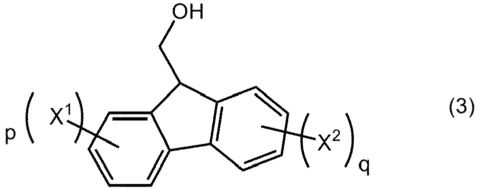

In formula (3), X¹ and X² are the same or different and are each a halogen atom.

The halogen atom is preferably a fluorine atom (F), a chlorine atom (Cl), a bromine atom (Br), or an iodine atom (I).

In formula (3), X¹ and X² are each preferably an iodine atom.

In formula (3), p is an integer of 1 to 4.

In formula (3), q is an integer of 0 to 4.

The amine-protecting group intermediate represented by formula (3) is preferably an amine-protecting group intermediate represented by the following formula (3a) or (3b).

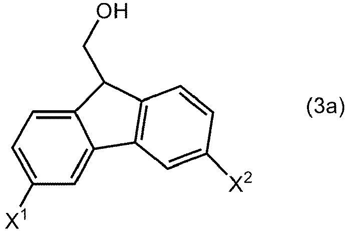

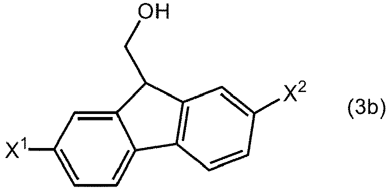

In formula (3a) or (3b), X¹ and X² are the same or different and are each a halogen atom.

The halogen atom is preferably a fluorine atom (F), a chlorine atom (Cl), a bromine atom (Br), or an iodine atom (I), and more preferably a bromine atom (Br) or an iodine atom (I).

In formula (3a) or (3b), X¹ and X² are each more preferably a bromine atom (Br) or an iodine atom (I), and particularly preferably an iodine atom (I).

The amine-protecting group intermediates of the present invention are useful for the production of amine-protecting groups.

### [3] Method for Producing Amine-Protecting Group Intermediate

The amine-protecting group intermediate represented by formula (3) of the present invention can be produced by the following method.

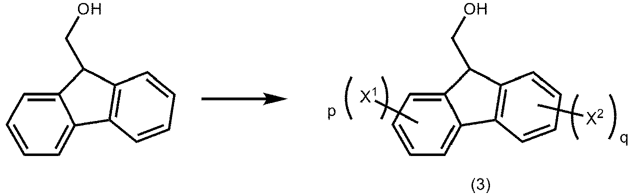

In formula (3), X¹ and X² are the same or different and are each a halogen atom.

9-Fluorenylmethanol is dissolved in 1,4-dioxane, chloroform (CHCl₃, trichloromethane), acetonitrile (AN), water (H₂O), or the like, and iodobenzene bis(trifluoroacetate) (PIFA, PhI(OCOCF₃)₂), iron trichloride (FeCl₃), or the like is added, followed by stirring at a temperature in the range of about 0°C to 100°C (preferably at room temperature) for a reaction time of about 10 minutes to 24 hours.

Then, iodine, bromine, chlorine, or fluorine is added, followed by stirring at a temperature in the range of about 0°C to 100°C (preferably at room temperature) for a reaction time of about 10 minutes to 24 hours.

After concentration, hexane is added and suspended, followed by filtration.

After the above operation, amine-protecting group intermediates, such as 2,7-diiodo-9-fluorenylmethanol (an example of formula (3b)) and 3,6-dibromo-9-fluorenylmethanol (an example of formula (3a)), can be obtained.

### (1) Production Example 1 of Amine-Protecting Group Intermediate Method for Producing 2,7-Diiodo-9-Fluorenylmethanol

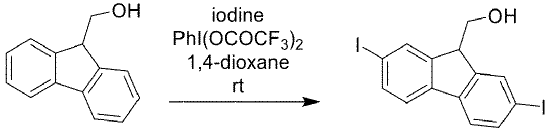

9-Fluorenylmethanol is dissolved in 1,4-dioxane, and iodobenzene bis(trifluoroacetate) (PIFA, PhI(OCOCF₃)₂) is added, followed by stirring at room temperature.

Then, iodine is added, followed by stirring at room temperature.

After concentration, hexane can be added and suspended, followed by filtration, thereby obtaining 2,7-diiodo-9-fluorenylmethanol.

### [4] Method for Producing Amine-Protecting Group

The amine-protecting group represented by formula (1) of the present invention can be produced by the following method.

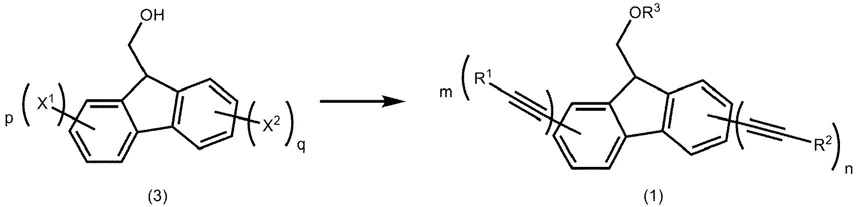

In formula (1), R¹ and R² are the same or different and are each a hydrogen atom, a halogen atom, an alkyl group, a trimethylsilyl group, an acetyl group, a benzoyl group, an ester group, an alkyl ester group, an aryl group, an alkyl alcohol group, a nitrile group, 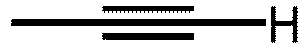 or a group represented by

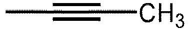

In formula (1), R³ is a hydrogen atom, a halogenated carbonyl group, or an active ester group.

In formula (1), m is an integer of 1 to 4.

In formula (1), n is an integer of 0 to 4.

In formula (3), X¹ and X² are the same or different and are each a halogen atom.

### Step A: Reaction to Introduce Groups with Triple Bond (-C≡C-R¹ and -C≡C-R² in Formula (1)) into Fluorenyl Compound

Groups with a triple bond can be introduced preferably by subjecting a fluorenyl compound to a coupling reaction.

Compounds used to introduce groups with a triple bond are preferably propyne (H-C≡C-CH₃), 1-(trimethylsilyl)-1-propyne, TMS acetylene (H-C≡C-TMS) (TMS: trimethylsilyl group (-Si(CH₃)₃)), bis(trimethylsilyl)acetylene, TMS-1,3-dibutyne (H-C≡C-C≡C-Si(CH₃)₃), butane-1,3-diyne (H-C≡C-C≡C-H), silver acetylide (Ag-C≡C-Ag), copper acetylide (Cu-C≡C-Cu), TMS-C≡C-C≡C-TMS, H-C≡C-C≡C-TMS, and the like.

In the coupling reaction, a palladium catalyst, a copper catalyst (Cu), or the like is preferably used.

The reaction temperature is preferably -20°C to 100°C, and more preferably 0°C to 50°C.

The reaction time is preferably 1 hour to 24 hours, and more preferably 1 hour to 10 hours.

A copper catalyst (Cu) or the like can also be preferably used in place of the palladium catalyst (Pd-cat.).

### Step B: Reaction to Remove Trimethylsilyl Groups (TMS, -Si(CH₃)₃) Introduced into Groups with Triple Bond (-C≡C-R¹ and -C≡C-R² in Formula (1)) of Fluorenyl Compound

A reaction can be performed to remove TMS groups introduced into the groups with a triple bond of the fluorenyl compound.

In a solvent, such as tetrahydrofuran (THF), methanol (MeOH), or water (H₂O), tetra-n-butylammonium fluoride (TBAF) (THF solution), potassium fluoride (KF), potassium carbonate (K₂CO₃), potassium hydroxide (KOH), or the like is preferably added to the fluorenyl compound having groups with a triple bond containing TMS groups to remove TMS.

The reaction temperature is preferably 0°C to 100°C.

The reaction time is preferably 1 hour to 48 hours.

### Step C: Reaction to Subject Fluorenyl Compound to Chloroformate Esterification (Reaction to Introduce Halogenated Carbonyl Group (R³ in Formula (1)) into Fluorenyl Compound)

The fluorenyl compound can be preferably subjected to chloroformate esterification to obtain a 9-fluorenylmethyloxycarbonyl protecting group (an amine-protecting group with an activated hydroxyl group).

In a solvent, such as dichloromethane (DCM), chloroform (CHCl₃), toluene, or tetrahydrofuran (THF), triphosgene (bis(trichloromethyl) carbonate), trichloromethyl chloroformate, dichloromethane, or the like is preferably added to the fluorenyl compound to perform chloroformate esterification. A halogenated carbonyl group (R³ in formula (1)) is introduced into the fluorenyl compound.

The reaction temperature is preferably -20°C to 50°C.

The reaction time is preferably 1 hour to 10 hours.

### Step D: Reaction to Introduce Active Ester Group (R³ in Formula (1)) into Fluorenyl Compound

When an N-succinimide ester group is introduced, N-hydroxysuccinimide and pyridine are dissolved in tetrahydrofuran (THF), and a fluorenyl compound (9-fluorenylmethyloxycarbonyl protecting group, Fmoc-chloride compound)/tetrahydrofuran (THF) solution is added to the reaction liquid, followed by stirring for minutes.

The reaction temperature is preferably -20°C to 50°C (e.g., 0°C or room temperature).

The reaction time is preferably 10 minutes to 24 hours (e.g., overnight).

After the above operation, an amine-protecting group can be obtained.

When another active ester group, such as a 4-nitrophenyl ester group, a 2,4-dinitrophenyl ester group, a pentafluorophenyl ester group, an N-hydroxyphthalimide ester group, or a 1-hydroxybenzotriazole ester group, is introduced, the reaction may be performed in the same manner using, as needed, a solvent other than THF in place of N-hydroxysuccinimide, and using 4-nitrophenol (DCM etc.), 2,4-nitrophenol (DCM etc.), pentafluorophenol (triethylamine (TEA), diethyl ether, etc.), N-hydroxyphthalimide (dicyclohexylamine (DCHA), an acetone/chloroform mixed solvent, etc.), or 1-hydroxybenzotriazole (pyridine etc.).

### (1) Production Example 1 of Amine-Protecting Group

### Production of 3,6-Di(1-Propynyl)-9-Fluorenylmethanol

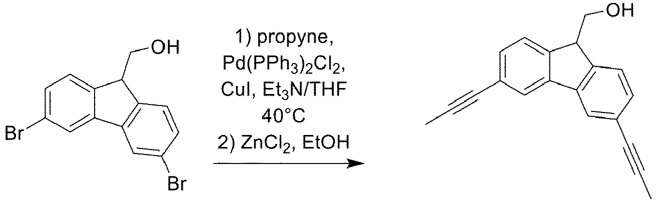

3,6-Dibromo-9-fluorenylmethanol, copper(I) iodide (CuI), and bis(triphenylphosphine)palladium(II) dichloride (Pd(PPh₃)₂Cl₂) are dissolved in tetrahydrofuran (THF), and triethylamine (Et₃N) and propyne (THF solution) are added, followed by stirring at 45°C for 1.5 hours.

After concentration, ethanol and zinc chloride (ZnCl₂) are added, followed by stirring at room temperature overnight.

Then, Celite filtration (washing with ethanol) and concentration are performed.

Then, the residue can be purified by silica gel column chromatography, thereby obtaining 3,6-di(1-propynyl)-9-fluorenylmethanol.

### Production of 3,6-Di(1-Propynyl)-Fmoc-Chloride

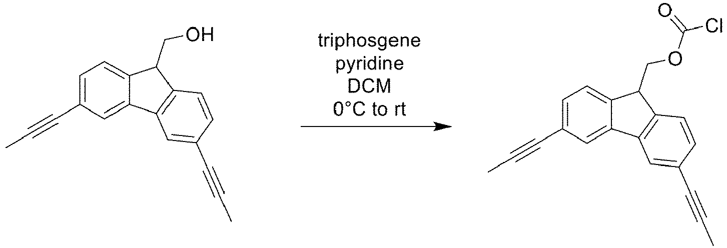

Pyridine and a dichloromethane (DCM) solution of 3,6-di(1-propynyl)-9-fluorenylmethanol are slowly added dropwise at 0°C to a dichloromethane (DCM) solution of triphosgene, followed by stirring at 0°C for 30 minutes.

Then, water is added, followed by extraction with dichloromethane (DCM), and the organic layer is dried over magnesium sulfate (MgSO₄).

After concentration, 3,6-di(1-propynyl)-Fmoc-chloride can be obtained.

### (2) Production Example 2 of Amine-Protecting Group

### Production of 3,6-Di(Trimethylsilylethynyl)-9-Fluorenylmethanol

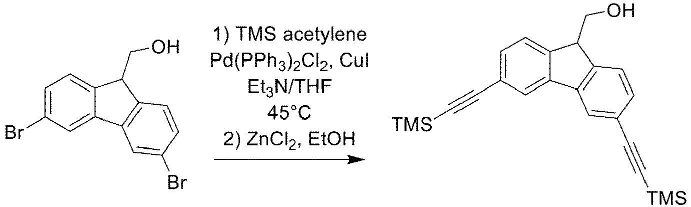

3,6-Dibromo-9-fluorenylmethanol, copper(I) iodide (CuI), and bis(triphenylphosphine)palladium(II) dichloride (Pd(PPh₃)₂Cl₂) are dissolved in tetrahydrofuran (THF), and triethylamine (Et₃N) and trimethylsilylacetylene (TMSA) are added, followed by stirring at 45°C for 1.5 hours.

After concentration, ethanol and zinc chloride (ZnCl₂) are added, followed by stirring at room temperature overnight.

Then, Celite filtration (washing with ethanol) and concentration are performed.

Then, the residue can be purified by silica gel column chromatography, thereby obtaining 3,6-di(trimethylsilylethynyl)-9-fluorenylmethanol.

### Production of 3,6-Di(Trimethylsilylethynyl)-Fmoc-Chloride

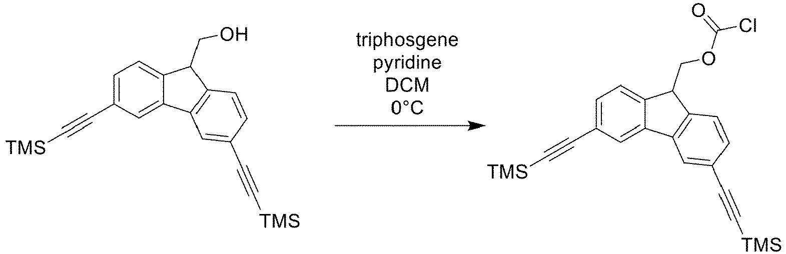

Pyridine and a dichloromethane (DCM) solution of 3,6-di(trimethylsilylethynyl)-9-fluorenylmethanol are slowly added dropwise at 0°C to a dichloromethane (DCM) solution of triphosgene, followed by stirring at 0°C for 30 minutes.

Then, water is added, followed by extraction with dichloromethane (DCM), and the organic layer is dried over magnesium sulfate (MgSO₄).

After concentration, 3,6-di(trimethylsilylethynyl)-Fmoc-chloride can be obtained.

### (3) Production Example 3 of Amine-Protecting Group

### Production of 2,7-Di(1-Propynyl)-9-Fluorenylmethanol

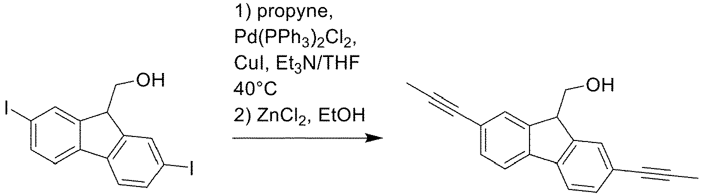

2,7-Diiodo-9-fluorenylmethanol, copper(I) iodide (CuI), and bis(triphenylphosphine)palladium(II) dichloride (Pd(PPh₃)₂Cl₂) are dissolved in tetrahydrofuran (THF), and triethylamine (Et₃N) and propyne (THF solution) are added, followed by stirring at 40°C for 4 hours.

After concentration, ethanol and zinc chloride (ZnCl₂) are added, followed by stirring at room temperature overnight.

Then, Celite filtration (washing with ethanol) and concentration are performed.

Then, the residue is purified by silica gel column chromatography to obtain a solid.

Then, diethyl ether and hexane can be added to the solid for crystallization, followed by filtration (washing with an excess amount of hexane), thereby obtaining 2,7-di(1-propynyl)-9-fluorenylmethanol.

### Production of 2,7-Di(1-Propynyl)-Fmoc-Chloride

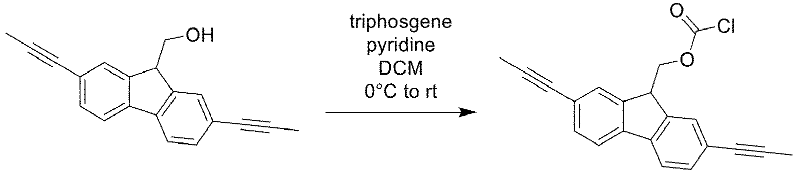

Pyridine and a dichloromethane (DCM) solution of 2,7-di(1-propynyl)-9-fluorenylmethanol are slowly added dropwise at 0°C to a dichloromethane (DCM) solution of triphosgene, followed by stirring at room temperature for 2 hours.

Then, water is added, followed by extraction with dichloromethane (DCM), and the organic layer is dried over magnesium sulfate (MgSO₄).

After concentration, 2,7-di(1-propynyl)-Fmoc-chloride can be obtained.

### Production of 2,7-Di(1-Propynyl)-Fmoc-N-Hydroxysuccinimide Ester

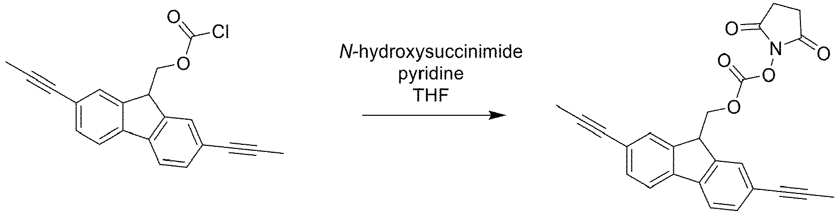

2,7-Di(1-propynyl)-Fmoc-chloride and N-hydroxysuccinimide are dissolved in tetrahydrofuran (THF), and a pyridine/tetrahydrofuran (THF) solution is added to the reaction liquid, followed by stirring at room temperature overnight.

Then, water is added to the reaction liquid, followed by extraction with ethyl acetate and drying over magnesium sulfate (MgSO₄), and the solvent is distilled off.

Then, the obtained residue can be purified by silica gel column chromatography (hexane/ethyl acetate = 4/1 -> 1/1), thereby obtaining 2,7-di(1-propynyl)-Fmoc-N-hydroxysuccinimide ester.

### (4) Production Example 4 of Amine-Protecting Group

### Production of 2,7-Di(Trimethylsilylethynyl)-9-Fluorenylmethanol

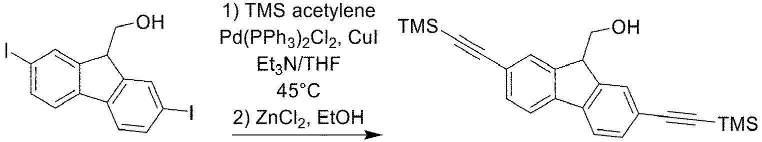

2,7-Diiodo-9-fluorenylmethanol, copper(I) iodide (CuI), and bis(triphenylphosphine)palladium(II) dichloride (Pd(PPh₃)₂Cl₂) are dissolved in tetrahydrofuran (THF), and triethylamine (Et₃N) and trimethylsilylacetylene (TMSA) are added, followed by stirring at 45°C for 1.5 hours.

After concentration, ethanol and zinc chloride (ZnCl₂) are added, followed by stirring at room temperature overnight.

Then, Celite filtration (washing with ethanol) and concentration are performed.

Then, the residue can be purified by silica gel column chromatography, thereby obtaining 2,7-di(trimethylsilylethynyl)-9-fluorenylmethanol.

### Production of 2,7-Di(Trimethylsilylethynyl)-Fmoc-Chloride

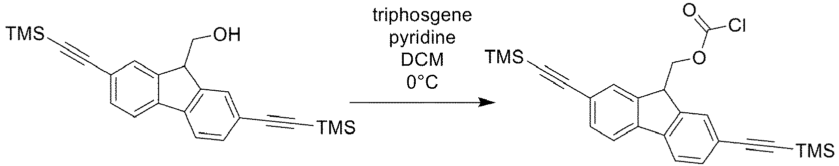

Pyridine and a dichloromethane (DCM) solution of 2,7-di(trimethylsilylethynyl)-9-fluorenylmethanol are slowly added dropwise at 0°C to a dichloromethane (DCM) solution of triphosgene, followed by stirring at 0°C for 30 minutes.

Then, water is added, followed by extraction with dichloromethane (DCM), and the organic layer is dried over magnesium sulfate (MgSO₄).

After concentration, 2,7-di(trimethylsilylethynyl)-Fmoc-chloride can be obtained.

### Production of 2,7-Di(Trimethylsilylethynyl)-Fmoc-N-Hydroxysuccinimide Ester

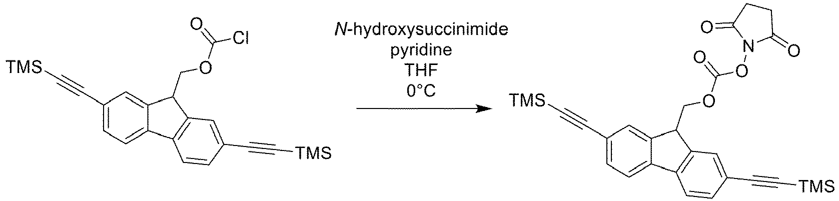

N-hydroxysuccinimide and pyridine are dissolved in tetrahydrofuran (THF), and a tetrahydrofuran (THF) solution of 2,7-di(trimethylsilylethynyl)-Fmoc-chloride is added at 0°C to the reaction liquid, followed by stirring at the same temperature for 45 minutes.

Then, water is added to the reaction liquid, followed by extraction with ethyl acetate and drying over magnesium sulfate (MgSO₄), and the solvent is distilled off.

Then, diethyl ether and hexane can be added to the obtained residue, and the resulting solid can be filtered, followed by purification by silica gel column chromatography, thereby obtaining 2,7-di(trimethylsilylethynyl)-Fmoc-N-hydroxysuccinimide ester.

### (5) Production 5 of Amine-Protecting Group

### Production of 2,7-Di(Phenylethynyl)-9-Fluorenylmethanol

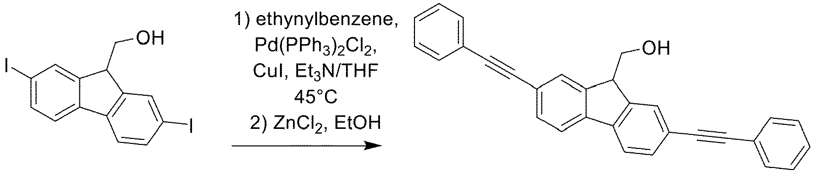

2,7-Diiodo-9-fluorenylmethanol, copper(I) iodide (CuI), and bis(triphenylphosphine)palladium(II) dichloride (Pd(PPh₃)₂Cl₂) are dissolved in tetrahydrofuran (THF), and triethylamine (Et₃N) and ethynylbenzene (phenyl acetylene) are added, followed by stirring at 45°C for 2.5 hours.

After concentration, ethanol and zinc chloride (ZnCl₂) are added, followed by stirring at room temperature overnight.

Then, Celite filtration (washing with ethanol) and concentration are performed.

Then, the residue can be purified by silica gel column chromatography, thereby obtaining 2,7-di(phenylethynyl)-9-fluorenylmethanol.

### Production of 2,7-Di(Phenylethynyl)-Fmoc-Chloride

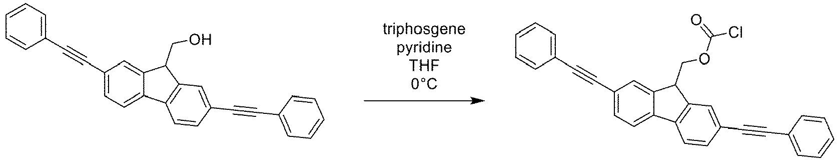

Pyridine and a tetrahydrofuran (THF) solution of 2,7-di(phenylethynyl)-9-fluorenylmethanol are slowly added dropwise at 0°C to a tetrahydrofuran (THF) solution of triphosgene, followed by stirring at 0°C for 20 minutes.

Then, triphosgene and pyridine are added, followed by stirring at 0°C for 1.5 hours.

Then, water is added, followed by extraction with ethyl acetate, and the organic layer is dried over magnesium sulfide (MgS) .

After concentration, 2,7-di(phenylethynyl)-Fmoc-chloride can be obtained.

### Production of 2,7-Di(Phenylethynyl)-Fmoc-N-Hydroxysuccinimide Ester

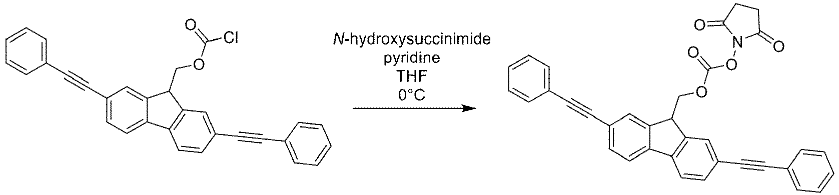

N-hydroxysuccinimide and pyridine are dissolved in tetrahydrofuran (THF), and a 2,7-di(phenylethynyl)-Fmoc-chloride/tetrahydrofuran (THF) solution is added at 0°C to the reaction liquid, followed by stirring at the same temperature for 20 minutes.

Then, water is added to the reaction liquid, followed by extraction with ethyl acetate and drying over magnesium sulfide (MgS), and the solvent is distilled off.

Then, the obtained residue can be purified by silica gel column chromatography (hexane/ethyl acetate = 2/1 -> 5/2), thereby obtaining 2,7-di(phenylethynyl)-Fmoc-N-hydroxysuccinimide ester.

### (6) Production 6 of Amine-Protecting Group

### Production of 2,7-Di(Cyclopropylethynyl)-9-Fluorenylmethanol

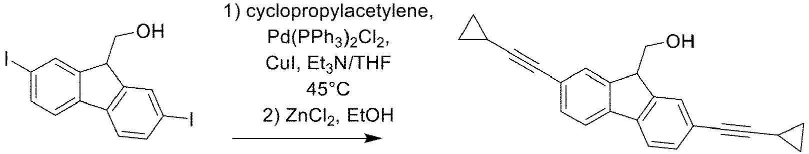

2,7-Diiodo-9-fluorenylmethanol, copper(I) iodide (CuI), and bis(triphenylphosphine)palladium(II) dichloride (Pd(PPh₃)₂Cl₂) are dissolved in tetrahydrofuran (THF), and triethylamine (Et₃N) and cyclopropylacetylene (ethynylcyclopropane) are added, followed by stirring at 45°C for 2 hours.

After concentration, ethanol and zinc chloride (ZnCl₂) are added, followed by stirring at room temperature overnight.

Then, Celite filtration (washing with ethanol) and concentration are performed.

Then, the residue can be purified by silica gel column chromatography (hexane/ethyl acetate = 4/1 -> 3/1), thereby obtaining 2,7-di(cyclopropylethynyl)-9-fluorenylmethanol.

### Production of 2,7-Di(Cyclopropylethynyl)-Fmoc-Chloride

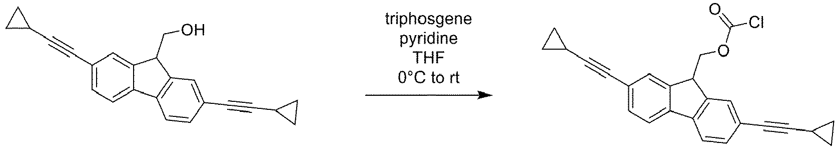

A tetrahydrofuran (THF) solution of 2,7-di(cyclopropylethynyl)-9-fluorenylmethanol and pyridine are added at 0°C to a tetrahydrofuran (THF) solution of triphosgene, followed by stirring at 0°C for 10 minutes and further stirring at room temperature for 30 minutes.

Then, water is added, followed by extraction with ethyl acetate, and the organic layer is dried over magnesium sulfide (MgS) .

After concentration, 2,7-di(cyclopropylethynyl)-Fmoc-chloride can be obtained.

### Production of 2,7-Di(Cyclopropylethynyl)-Fmoc-N-Hydroxysuccinimide Ester

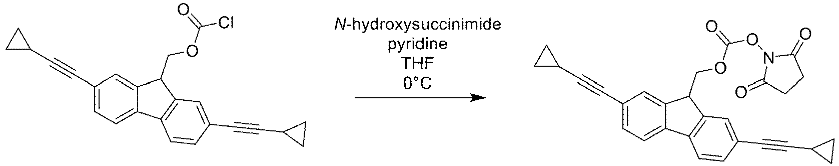

N-hydroxysuccinimide and pyridine are dissolved in tetrahydrofuran (THF), and a tetrahydrofuran (THF) solution of 2,7-di(cyclopropylethynyl)-Fmoc-chloride is added at 0°C to the reaction liquid, followed by stirring at the same temperature for 20 minutes.

Then, water is added to the reaction liquid, followed by extraction with ethyl acetate and drying over magnesium sulfide (MgS), and the solvent is distilled off.

Then, hexane can be added to the obtained residue for crystallization, followed by filtration, thereby obtaining 2,7-di(cyclopropylethynyl)-Fmoc-N-hydroxysuccinimide ester.

### (7) Production 7 of Amine-Protecting Group

### Production of 2,7-Di(Ethynyl)-9-Fluorenylmethanol

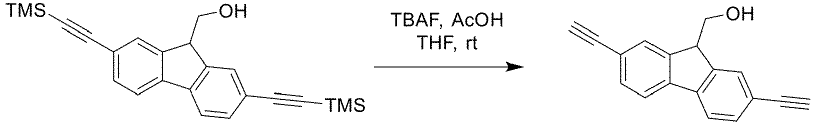

Acetic acid and tetra-n-butylammonium fluoride (TBAF, THF solution) are added to a tetrahydrofuran (THF) solution of 2,7-di(trimethylsilylethynyl)-9-fluorenylmethanol, followed by stirring at room temperature for 10 minutes.

After concentration, water and ethyl acetate are added for extraction, the organic layer is then dried over magnesium sulfide (MgS), and the solvent is distilled off.

The residue can be purified by silica gel column chromatography (hexane/ethyl acetate = 9/1 -> 2/1), thereby obtaining 2,7-di(ethynyl)-9-fluorenylmethanol.

### Production of 2,7-Di(Ethynyl)-Fmoc-Chloride

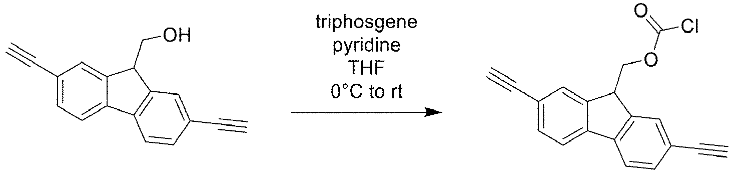

A tetrahydrofuran (THF) solution of 2,7-di(ethynyl)-9-fluorenylmethanol and pyridine are added at 0°C to a tetrahydrofuran (THF) solution of triphosgene, followed by stirring at 0°C for 45 minutes.

Then, water is added, followed by extraction with ethyl acetate, and the organic layer is dried over magnesium sulfide (MgS) .

After concentration, 2,7-di(ethynyl)-Fmoc-chloride can be obtained.

### Production of 2,7-Di(Ethynyl)-Fmoc-N-Hydroxysuccinimide Ester

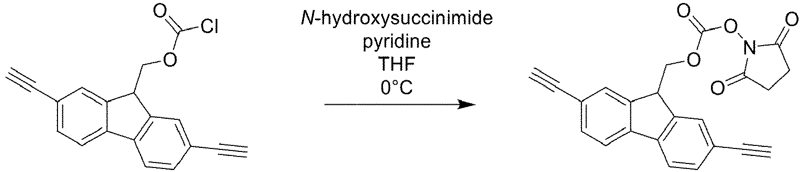

N-hydroxysuccinimide and pyridine are dissolved in tetrahydrofuran (THF), and a 2,7-di(ethynyl)-Fmoc-chloride/tetrahydrofuran (THF) solution is added at 0°C to the reaction liquid, followed by stirring at the same temperature for 15 minutes.

Then, water is added to the reaction liquid, followed by extraction with ethyl acetate and drying over magnesium sulfide (MgS), and the solvent is distilled off.

Then, hexane can be added to the obtained residue for crystallization, followed by filtration, thereby obtaining 2,7-di(ethynyl)-Fmoc-N-hydroxysuccinimide ester.

The embodiments of the present invention are described above; however, the present invention is not limited to these examples. Needless to say, the present invention can be implemented in various forms within the scope not departing from the gist of the present invention.

### Examples

Embodiments of the present invention are described in more detail below based on Examples.

The present invention is not limited thereto.

### Examples

### (1) Production 1 of Amine-Protecting Group

### (1-1) Production of 3,6-Di(1-Propynyl)-9-Fluorenylmethanol

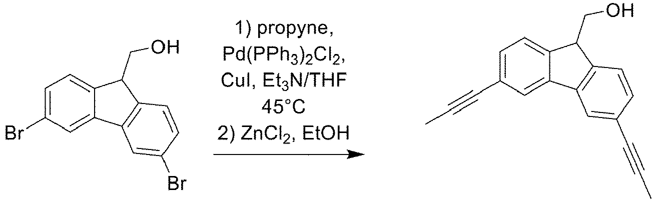

3,6-Dibromo-9-fluorenylmethanol (5.00 g, 11.6 mmol), copper(I) iodide (CuI) (221 mg, 1.16 mmol), and bis(triphenylphosphine)palladium(II) dichloride (Pd(PPh₃)₂Cl₂) (814 mg, 1.16 mmol) were dissolved in tetrahydrofuran (THF) (35 mL), and triethylamine (Et₃N) (7.8 mL) and propyne (THF solution, 1M in THF) (72.5 mL, 72.5 mmol) were added, followed by stirring at 45°C for 1.5 hours.

After concentration, ethanol (50 mL) and zinc chloride (ZnCl₂) (634 mg, 4.64 mmol) were added, followed by stirring at room temperature overnight.

Then, Celite filtration (washing with ethanol) and concentration were performed.

Then, the residue was purified by silica gel column chromatography, thereby obtaining 3,6-di(1-propynyl)-9-fluorenylmethanol (3.67 g, 9.43 mmol, 810) as a yellow solid.

### NMR

¹H NMR (400 MHz, CDCl₃): δ 7.78 (S, 2H), 7.51-7.56 (m, 2H), 7.36-7.7.38 (m, 2H), 4.01-4.11 (m, 1H), 4.00-4.02 (m, 2H), 2.11 (S, 6H)

### (2) Production 2 of Amine-Protecting Group

### (2-1) Production of 3,6-Di(Trimethylsilylethynyl)-9-Fluorenylmethanol

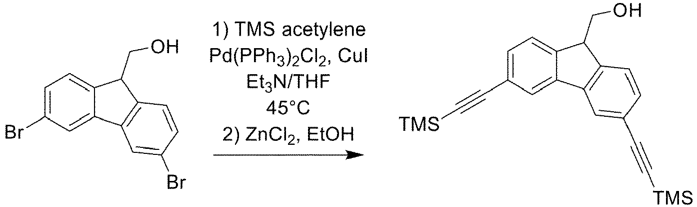

3,6-Dibromo-9-fluorenylmethanol (5.00 g, 11.6 mmol), copper(I) iodide (CuI) (221 mg, 1.16 mmol), and bis(triphenylphosphine)palladium(II) dichloride (Pd(PPh₃)₂Cl₂) (814 mg, 1.16 mmol) were dissolved in tetrahydrofuran (THF) (35 mL), and triethylamine (Et₃N) (7.8 mL) and trimethylsilylacetylene (TMSA) (6.4 mL, 46.4 mmol) were added, followed by stirring at 45°C for 1.5 hours.

After concentration, ethanol (50 mL) and zinc chloride (ZnCl₂) (634 mg, 4.64 mmol) were added, followed by stirring at room temperature overnight.

Then, Celite filtration (washing with ethanol) and concentration were performed.

Then, the residue was purified by silica gel column chromatography, thereby obtaining 3,6-di(trimethylsilylethynyl)-9-fluorenylmethanol (3.67 g, 9.43 mmol, 810) as a yellow solid.

### NMR

¹H-NMR (CDCl₃): δ 7.74-7.67 (m, 4H), 7.55-7.52 (m, 2H), 4.11-4.02 (m, 3H), 0.29 (s, 18H)
¹³C-NMR (CDCl₃): δ 144.8, 141.2, 131.9, 128.4, 122.1, 120.3, 105.6, 95.0, 65.0, 50.2, 0.2

### (2-2) Production of 3,6-Di(Trimethylsilylethynyl)-Fmoc-Chloride

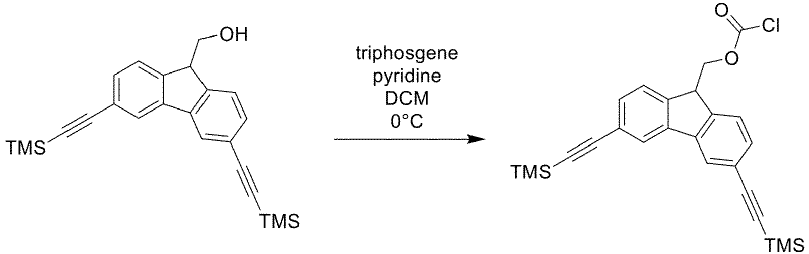

Pyridine (311 µL, 3.86 mmol) and a dichloromethane (DCM) (2.0 mL) solution of 3,6-di(trimethylsilylethynyl)-9-fluorenylmethanol (1.00 g, 2.57 mmol) were slowly added dropwise at 0°C to a dichloromethane (DCM) (5.0 mL) solution of triphosgene (419 mg, 1.41 mmol), followed by stirring at 0°C for 30 minutes.

Then, water was added, followed by extraction with dichloromethane (DCM), and the organic layer was dried over magnesium sulfate (MgSO₄).

After concentration, 3,6-di(trimethylsilylethynyl)-Fmoc-chloride (1.14 g, 2.53 mmol, 98%) was obtained as a yellow solid.

### NMR

¹H-NMR (CDCl₃): δ 7.70-7.68 (m, 4H), 7.58-7.55 (m, 2H), 4.56 (d, 2H, J = 6.9 Hz), 4.26 (t, 1H, J = 6.9 Hz), 0.30 (s, 18H)
¹³C-NMR (CDCl₃): δ 150.9, 142.8, 140.9, 132.5, 128.6, 122.6, 120.5, 105.2, 95.5, 72.7, 46.1, 0.1

### (3) Production 1 of Amine-Protecting Group Intermediate

### (3-1) Production of 2,7-Diiodo-9-Fluorenylmethanol

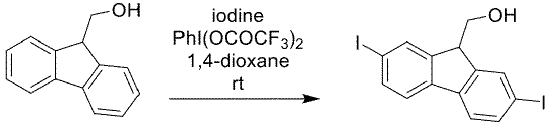

9-Fluorenylmethanol (1.02 g, 5.20 mmol) was dissolved in 1,4-dioxane (5.2 mL), and iodobenzene bis(trifluoroacetate) (PIFA, PhI(OCOCF₃)₂) (2.68 g, 6.24 mmol) was added, followed by stirring at room temperature for 10 minutes.

Then, iodine (1.32 g, 5.20 mmol) was added, followed by stirring at room temperature for 1 hour.

After concentration, hexane (10 mL) was added and suspended, followed by filtration, thereby obtaining 2,7-diiodo-9-fluorenylmethanol (2.17 g, 4.84 mmol, 93%) as a beige solid.

### NMR

¹H-NMR (CDCl₃): δ7.95-7.94 (m, 2H), 7.75-7.71 (m, 2H), 7.49-7.47 (m, 2H), 4.06 (t, 1H, J = 5.7 Hz), 4.00 (d, 2H, J = 5.7 Hz),
¹³C-NMR (CDCl₃): δ 146.3, 140.3, 137.0, 134.1, 121.9, 93.0, 64.7, 50.1

### (4) Production 3 of Amine-Protecting Group

### (4-1) Production of 2,7-Di(1-Propynyl)-9-Fluorenylmethanol

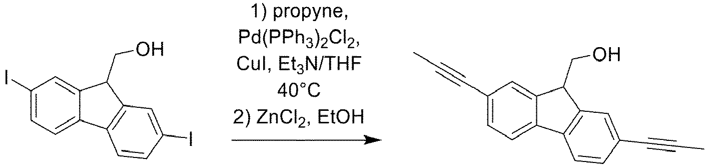

2,7-Diiodo-9-fluorenylmethanol (5.00 g, 11.6 mmol), copper(I) iodide (CuI) (663 mg, 3.48 mmol), and bis(triphenylphosphine)palladium(II) dichloride (Pd(PPh₃)₂Cl₂) (2.44 g, 3.48 mmol) were dissolved in tetrahydrofuran (THF) (25 mL), and triethylamine (Et₃N) (7.75 mL) and propyne (1M in THF solution) (72.5 mL, 72.5 mmol) were added, followed by stirring at 40°C for 4 hours.

After concentration, ethanol (50 mL) and zinc chloride (1.90 g, 13.92 mmol) were added, followed by stirring at room temperature overnight.

Then, Celite filtration (washing with ethanol) and concentration were performed.

Then, the residue was purified by silica gel column chromatography to obtain a yellow to brown solid (3.30 g, 12.1 mmol, quant.).

Then, diethyl ether and hexane were added to the solid for crystallization, followed by filtration (washing with an excess amount of hexane), thereby obtaining 2,7-di(1-propynyl)-9-fluorenylmethanol (2.37 g, 8.70 mmol, 75%) as a yellow solid.

### NMR

¹H-NMR (CDCl₃): δ 7.65-7.62 (m, 4H), 7.44-7.41 (m, 2H), 4.05 (t, 1H, J = 5.7 Hz), 3.99 (d, 2H, J = 5.7 Hz), 2.08 (s, 6H)
¹³C-NMR (CDCl₃): δ 144.7, 140.5, 131.3, 128.0, 122.9, 120.1, 86.5, 80.3, 65.1, 50.1, 4.6

### (4-2) Production of 2,7-Di(1-Propynyl)-Fmoc-Chloride

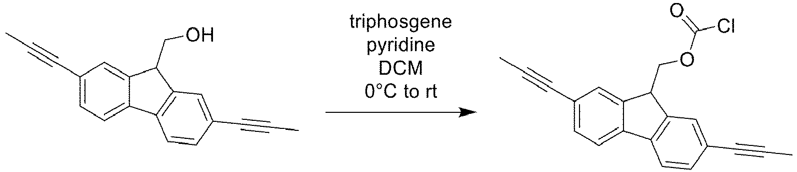

2,7-Di(1-propynyl)-9-fluorenylmethanol (1.41 g, 5.18 mmol) and a dichloromethane (DCM) (3.0 mL) solution of pyridine (626 µL, 7.77 mmol) were slowly added dropwise at 0°C to a dichloromethane (DCM) (11.0 mL) solution of triphosgene (767 mg, 2.59 mmol), followed by stirring at room temperature for 2 hours.

Then, water was added, followed by extraction with dichloromethane (DCM), and the organic layer was dried over magnesium sulfate (MgSO₄).

After concentration, 2,7-di(1-propynyl)-Fmoc-chloride (1.61 g, 4.81 mmol, 93%) was obtained as a brown solid.

### NMR

¹H-NMR (CDCl₃): δ 7.66-7.60 (m, 4H), 7.48-7.45 (m, 2H), 4.53 (d, 2H, J = 7.2 Hz), 4.24 (t, 1H, J = 7.2 Hz), 2.10 (s, 6H)
¹³C-NMR (CDCl₃): δ 150.9, 142.8, 140.2, 132.0, 128.1, 123.3, 120.3, 87.0, 80.0, 73.0, 46.1, 4.6

### (4-3) Production of 2,7-Di(1-Propynyl)-Fmoc-N-Hydroxysuccinimide Ester

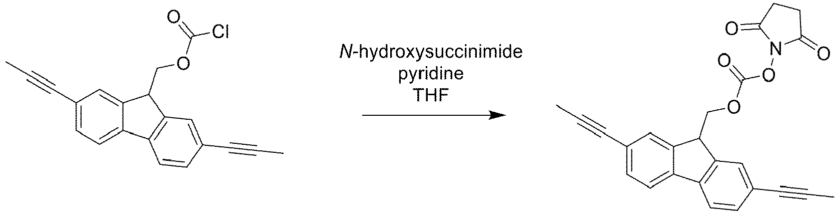

2,7-Di(1-propynyl)-Fmoc-chloride) (100 mg, 1.36 mmol) and N-hydroxysuccinimide (38.0 mg, 0.330 mmol) were dissolved in tetrahydrofuran (THF) (3.0 mL), and a tetrahydrofuran (THF) (1.0 mL) solution of pyridine (36.2 µL, 0.450 mmol) was added to the reaction liquid, followed by stirring at room temperature overnight.

Then, water was added to the reaction liquid, followed by extraction with ethyl acetate and drying over magnesium sulfate (MgSO₄), and the solvent was distilled off.

Then, the obtained residue was purified by silica gel column chromatography (hexane/ethyl acetate = 4/1 -> 1/1), thereby obtaining 2,7-di(1-propynyl)-Fmoc-N-hydroxysuccinimide ester (45.7 mg, 0.111 mmol, 37%) as a brown solid.

### NMR

¹H-NMR (CDCl₃): δ 7.65-7.62 (m, 4H), 7.47-7.44 (m, 2H), 4.53 (d, 2H, J = 7.2 Hz), 4.27 (t, 1H, J = 7.2 Hz), 2.85 (s, 4H), 2.08 (s, 6H)
¹³C-NMR (CDCl₃): δ 168.9, 151.9, 143.0, 140.5, 132.2, 128.6, 123.6, 120.5, 87.2, 80.3, 72.9, 46.5, 25.8, 4.8

### (5) Production 1 of Special Amino Acid

### (5-1) Production of 2,7-Di(1-Propynyl)-Fmoc-Gly-OH

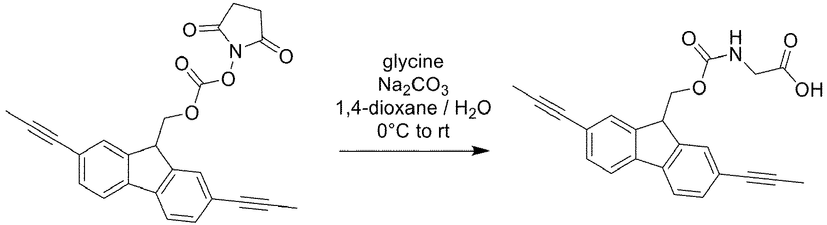

H-Gly-OH (1.319 g, 17.57 mmol) was added to a 10% sodium carbonate aqueous solution (50 mL), followed by stirring at 0°C.

Then, a 1,4-dioxane (50 mL) solution of 2,7-di(1-propynyl)-Fmoc-N-hydroxysuccinimide ester (5.35 g, 15.98 mmol) was slowly added dropwise at 0°C to this solution, followed by stirring at 0°C for 30 minutes and further stirring at room temperature for 4 hours.

Then, ethyl acetate, water, and 1N hydrochloric acid were added to adjust the pH of the reaction liquid to 2, and the organic layer was extracted with ethyl acetate.

After distilling off the solvent, the residue was purified by preparative TLC (hexane/ethyl acetate = 1/1), thereby obtaining 2,7-di(1-propynyl)-Fmoc-Gly-OH (5.967 g, 15.98 mmol, quant) as a yellow solid.

### NMR

¹H-NMR (MeOD-d4): δ 7.70-7.64 (m, 4H), 7.39-7.36 (m, 2H), 4.32 (d, 2H, J = 6.9 Hz), 4.18 (t, 1H, J = 6.9 Hz), 3.85 (s, 2H), 2.04 (s, 6H)
¹³C-NMR (MeOD-d4): δ 173.6, 159.0, 145.5, 141.3, 132.3, 129.1, 124.4, 121.0, 86.8, 81.0, 68.1, 48.1, 43.1, 3.9

### (5-2) Production of 2,7-Di(1-Propynyl)-Fmoc-Ala-OH

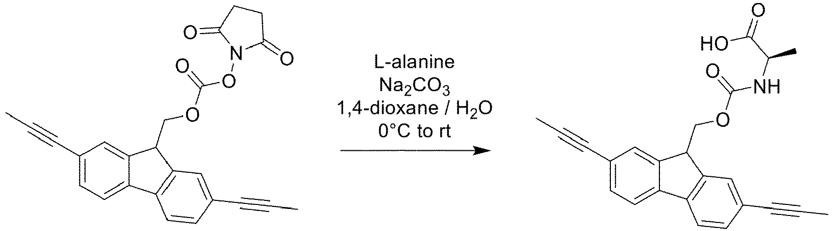

H-Phe-OH (25 mg, 0.15 mmol) was added to a 10% sodium carbonate aqueous solution (0.5 mL) and 1,4-dioxane (0.5 mL), followed by stirring at 0°C.

Then, a 1,4-dioxane (1.5 mL) solution of 2,7-di(1-propynyl)-Fmoc-N-hydroxysuccinimide ester (50 mg, 0.15 mmol) was slowly added dropwise at 0°C to this solution, followed by stirring at 0°C for 15 minutes and further stirring at room temperature for 15 minutes.

Then, ethyl acetate, water, and 1N hydrochloric acid were added to adjust the pH of the reaction liquid to 2, and the organic layer was extracted with ethyl acetate.

After distilling off the solvent, the residue was purified by preparative TLC (hexane/ethyl acetate = 1/1), thereby obtaining 2,7-di(1-propynyl)-Fmoc-Ala-OH (10.8 mg, 0.023 mmol, 160) as a yellow solid.

### NMR

¹H-NMR (MeOD-d4): δ 7.69-7.66 (m, 2H), 7.65-7.62 (m, 2H), 7.38-7.35 (m, 2H), 4.37-4.29 (m, 2H), 4.25-4.14 (m, 2H), 2.04 (s, 6H), 1.40 (d, 3H, J = 7.2 Hz)
¹³C-NMR (MeOD-d4): δ 175.2, 158.3, 145.7, 145.4, 141.4, 132.3, 129.1, 124.4, 121.0, 86.9, 80.9, 67.5, 50.9, 17.8, 3.9

### (5-3) Production of 2,7-Di(1-Propynyl)-Fmoc-Phe-OH

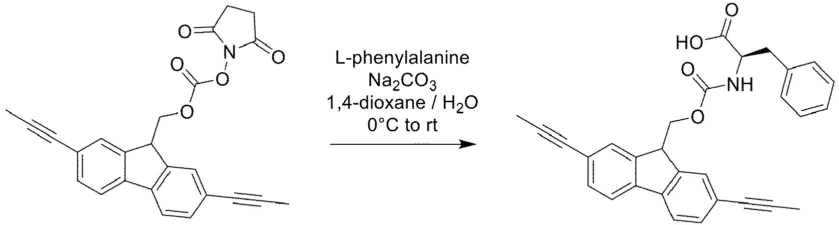

H-Phe-OH (25 mg, 0.15 mmol) was added to a 10% sodium carbonate aqueous solution (0.5 mL) and 1,4-dioxane (0.5 mL), followed by stirring at 0°C.

Then, a 1,4-dioxane (1.5 mL) solution of 2,7-di(1-propynyl)-Fmoc-N-hydroxysuccinimide ester (50 mg, 0.15 mmol) was slowly added dropwise at 0°C to this solution, followed by stirring at 0°C for 15 minutes and further stirring at room temperature for 15 minutes.

Then, ethyl acetate, water, and 1N hydrochloric acid were added to adjust the pH of the reaction liquid to 2, and the organic layer was extracted with ethyl acetate.

After distilling off the solvent, the residue was purified by preparative TLC (hexane/ethyl acetate = 1/1), thereby obtaining 2,7-di(1-propynyl)-Fmoc-Phe-OH (10.8 mg, 0.023 mmol, 160) as a yellow solid.

### NMR

¹H-NMR (CDCl₃): δ 7.64-7.56 (m, 5H), 7.44-7.41 (m, 2H), 7.32-7.25 (m, 4H), 7.18-7.16 (m, 2H), 5.24 (d, 1H, J = 8.4 Hz), 4.72 (ddd, 1H, J = 5.4 Hz, 5.4 Hz, 8.4 Hz), 4.42 (dd, 1H, J = 6.9 Hz, 10.8 Hz), 4.28 (dd, 1H, J = 6.9 Hz, 10.8 Hz), 4.13 (t, 1H, J = 6.9 Hz), 3.22 (dd, 1H, J = 5.4 Hz, 14.1 Hz), 3.14 (dd, 1H, J = 5.4 Hz, 14.1 Hz)
¹³C-NMR (CDCl₃): δ 175.4, 155.7, 144.0, 140.3, 135.6, 131.5, 129.6, 128.9, 128.2, 127.4, 123.0, 120.1, 86.6, 80.3, 66.8, 54.7, 47.0, 38.0, 4.6

### (5-4) Production of 2,7-Di(1-Propynyl)-Fmoc-Ser(tBu)-OH

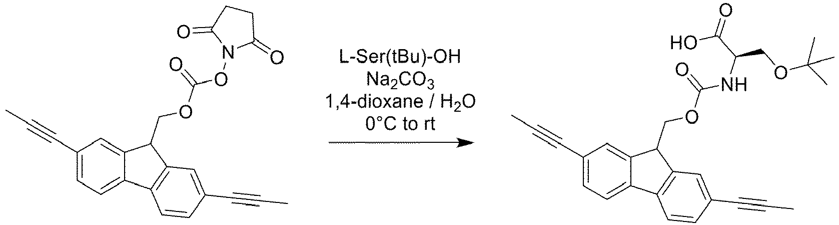

H-Ser(tBu)-OH (48.2 mg, 0.299 mmol) was added to a 10% sodium carbonate aqueous solution (0.5 mL) and 1,4-dioxane (0.5 mL), followed by stirring at 0°C.

Then, a 1,4-dioxane (3.0 mL) solution of 2,7-di(1-propynyl)-Fmoc-N-hydroxysuccinimide ester (100 mg, 0.299 mmol) was slowly added dropwise at 0°C to this solution, followed by stirring at 0°C for 15 minutes.

Then, ethyl acetate, water, and 1N hydrochloric acid were added to adjust the pH of the reaction liquid to 2, and the organic layer was extracted with ethyl acetate.

After distilling off the solvent, the residue was purified by preparative TLC (hexane/ethyl acetate = 1/1), thereby obtaining 2,7-di(1-propynyl)-Fmoc-Ser(tBu)-OH (16.8 mg, 0.037 mmol, 25%) as a white solid.

### NMR

¹H-NMR (CDCl₃): δ 7.64-7.60 (m, 4H), 7.44-7.41 (m, 2H), 5.66 (d, 1H, J = 7.5 Hz), 4.52-4.46 (ddd, 1H, J = 7.5 Hz, 5.4 Hz, 3.6 Hz), 4.36 (d, 2H, J = 6.9 Hz), 4.17 (t, 1H, J = 6.6 Hz), 3.92 (dd, 1H, J = 3.6 Hz, 9.0 Hz), 3.59 (dd, 1H, J = 5.4 Hz, 9.0 Hz), 2.07 (s, 6H), 1.22 (s, 9H)
¹³C-NMR (CDCl₃): δ 173.9, 156.1, 144.3, 144.0, 140.3, 131.5, 128.3, 122.9, 120.1, 86.5, 80.3, 74.8, 66.9, 61.7, 54.1, 47.0, 27.4, 4.6

### (5-5) Production of 2,7-Di(1-Propynyl)-Fmoc-Glu(tBu)-OH

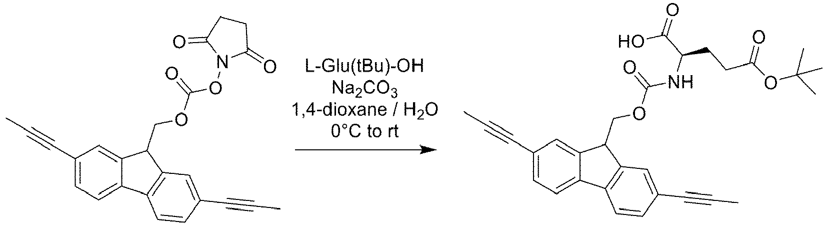

H-Lys(Boc)-OH (60.8 mg, 0.299 mmol) was added at 0°C to a 10% sodium carbonate aqueous solution (0.5 mL) and 1,4-dioxane (0.5 mL), followed by stirring.

Then, a 1,4-dioxane (3.0 mL) solution of 2,7-di(1-propynyl)-Fmoc-N-hydroxysuccinimide ester (100 mg, 0.299 mmol) was slowly added dropwise at 0°C to this solution, followed by stirring at 0°C for 15 minutes and further stirring at room temperature for 15 minutes.

Then, ethyl acetate, water, and 1N hydrochloric acid were added to adjust the pH of the reaction liquid to 2, and the organic layer was extracted with ethyl acetate.

After distilling off the solvent, the residue was purified by preparative TLC (hexane/ethyl acetate = 1/1), thereby obtaining 2,7-di(1-propynyl)-Fmoc-Glu(tBu)-OH (11.5 mg, 0.023 mmol, 150) as a yellow solid.

### NMR

¹H-NMR (CDCl₃): δ 7.63-7.58 (m, 4H), 7.43-7.40 (m, 2H), 5.59 (d, 1H, J = 7.8 Hz), 4.43-4.31 (m, 3H), 4.14 (t, 1H, J = 6.9 Hz), 2.53-2.34 (m, 2H), 2.29-2.19 (m, 1H), 2.07 (s, 6H), 2.07-1.96 (m, 1H), 1.45 (s, 9H)
¹³C-NMR (CDCl₃): δ 175.1, 172.8, 156.2, 144.0, 140.3, 131.5, 128.2, 123.0, 120.1, 86.6, 81.5, 80.3, 66.9, 53.6, 47.1, 31.8, 28.2, 27.6, 4.6

### (6) Production 4 of Amine-Protecting Group

### (6-1) Production of 2,7-Di(Trimethylsilylethynyl)-9-Fluorenylmethanol

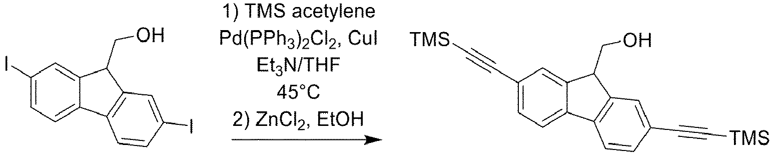

2,7-Diiodo-9-fluorenylmethanol (5.00 g, 11.6 mmol), copper(I) iodide (CuI) (221 mg, 1.16 mmol), and bis(triphenylphosphine)palladium(II) dichloride (Pd(PPh₃)₂Cl₂,) (814 mg, 1.16 mmol) were dissolved in tetrahydrofuran (THF) (35 mL), and triethylamine (Et₃N) (7.8 mL) and trimethylsilylacetylene (TMSA) (6.4 mL, 46.4 mmol) were added, followed by stirring at 45°C for 1.5 hours.

After concentration, ethanol (50 mL) and zinc chloride (ZnCl₂) (634 mg, 4.64 mmol) were added, followed by stirring at room temperature overnight.

Then, Celite filtration (washing with ethanol) and concentration were performed.

Then, the residue was purified by silica gel column chromatography, thereby obtaining 2,7-di(trimethylsilylethynyl)-9-fluorenylmethanol (3.67 g, 9.43 mmol, 810) as a yellow solid.

### NMR

¹H-NMR (CDCl₃): δ 7.72-7.65 (m, 4H), 7.53-7.50 (m, 2H), 4.07 (t, 1H, J = 6.6 Hz), 3.99 (d, 2H, J = 6.6 Hz), 0.27 (s, 18H)
¹³C-NMR (CDCl₃): δ 144.8, 141.2, 131.9, 128.5, 122.2, 120.3, 105.7, 95.0, 64.9, 50.2, -0.2

### (6-2) Production of 2,7-Di(Trimethylsilylethynyl)-Fmoc-Chloride

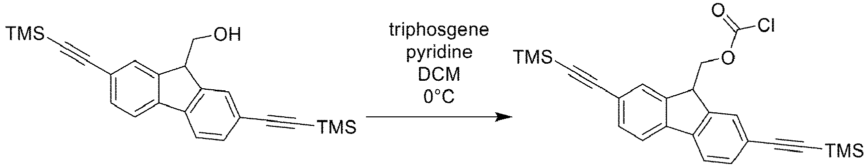

Pyridine (311 µL, 3.86 mmol) and a dichloromethane (DCM) (2.0 mL) solution of 2,7-di(trimethylsilylethynyl)-9-fluorenylmethanol (1.00 g, 2.57 mmol) were slowly added dropwise at 0°C to a dichloromethane (DCM) (5.0 mL) solution of triphosgene (419 mg, 1.41 mmol), followed by stirring at 0°C for 30 minutes.

Then, water was added, followed by extraction with dichloromethane (DCM), and the organic layer was dried over magnesium sulfate (MgSO₄).

After concentration, 2,7-di(trimethylsilylethynyl)-Fmoc-chloride (1.14 g, 2.53 mmol, 98%) was obtained as a yellow solid.

### NMR

¹H-NMR (CDCl₃): δ 7.69-7.66 (m, 4H), 7.56-7.53 (m, 2H), 4.55 (d, 2H, J = 7.2 Hz), 4.24 (t, 1H, J = 7.2 Hz), 0.28 (s, 18H)
¹³C-NMR (CDCl₃): δ 150.9, 142.8, 141.0, 132.6, 128.6, 122.6, 120.5, 105.2, 95.5, 72.8, 46.1, 0.1

### (6-3) Production of 2,7-Di(Trimethylsilylethynyl)-Fmoc-N-Hydroxysuccinimide Ester

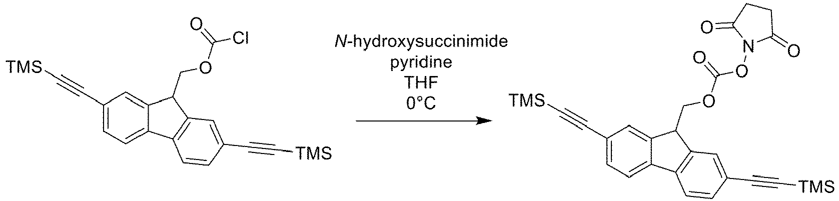

N-hydroxysuccinimide (2.43 g, 21.07 mmol) and pyridine (1.54 mL, 19.15 mmol) were dissolved in tetrahydrofuran (THF) (48 mL), and a tetrahydrofuran (THF) (48 mL) solution of 2,7-di(trimethylsilylethynyl)-Fmoc-chloride (8.64 g, 19.15 mmol) was added at 0°C to the reaction liquid, followed by stirring at the same temperature for 45 minutes.

Then, water was added to the reaction liquid, followed by extraction with ethyl acetate and drying over magnesium sulfate (MgSO₄), and the solvent was distilled off.

Then, diethyl ether (10 mL) and hexane (30 mL) were added to the obtained residue, and the generated solid was filtered, thereby obtaining 2,7-di(trimethylsilylethynyl)-Fmoc-N-hydroxysuccinimide ester (9.67 g, 18.24 mmol, 95%) as a yellow solid.

### NMR

¹H-NMR (CDCl₃): δ 7.71-7.67 (m, 4H), 7.57-7.54 (m, 2H), 4.56 (d, 2H, J = 7.2 Hz), 4.28 (t, 1H, J = 7.2 Hz), 2.87 (s, 4H), 0.28 (s, 18H)
¹³C-NMR (CDCl₃): δ 168.4, 151.5, 142.7, 140.8, 132.4, 128.6, 122.4, 120.3, 105.1, 95.3, 72.3, 46.2, 25.5, 0.0

### (7) Production 2 of Special Amino Acid

### (7-1) Production of 2,7-Di(Trimethylsilylethynyl)-Fmoc-Lys(Boc)-OH

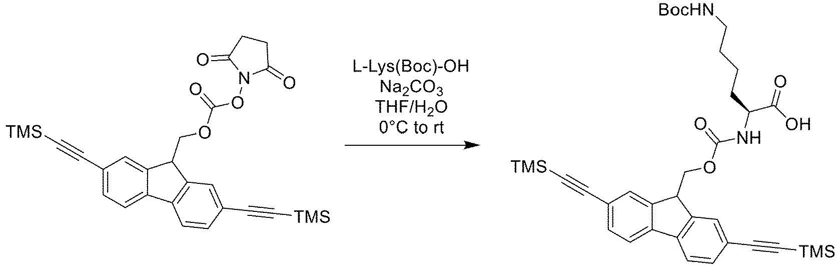

A tetrahydrofuran (THF) (1.5 mL) solution of 2,7-di(trimethylsilylethynyl)-Fmoc-N-hydroxysuccinimide ester (118 mg, 0.223 mmol) was added at 0°C to a water (0.5 mL)-tetrahydrofuran (THF) (0.5 mL) solution of H-Lys(Boc)-OH (57 mg, 0.233 mmol) and Na₂CO₃ (35 mg, 0.333 mmol), followed by stirring at 0°C for 1 hour.

Then, diethyl ether and water were added, 1N HCl was added to adjust the pH of the reaction liquid to 2, and the organic layer was extracted with diethyl ether (×2), dried over magnesium sulfate (MgSO₄), and then concentrated.

Then, hexane was added to the obtained residue for crystallization, followed by filtration (washing with hexane), thereby obtaining 2,7-di(trimethylsilylethynyl)-Fmoc-Lys(Boc)-OH (94.9 mg, 0.144 mmol, 65%) as a yellow solid.

### NMR

¹H-NMR (MeOD-d4): δ 7.70-7.63 (m, 4H), 7.42-7.40 (m, 2H), 4.42 (dd, 1H, J = 6.6 Hz, 10.8 Hz), 4.32 (dd, 1H, J = 6.6 Hz, 10.8 Hz), 4.14 (t, 1H, J = 6.6 Hz), 3.99 (dd, 1H, J = 8.1 Hz, 7.8 Hz), 3.03-2.99 (m, 2H), 1.87-1.75 (m, 1H), 1.69-1.57 (m, 1H), 1.50-1.32 (m, 13H), 0.25 (s, 18H)
¹³C-NMR (MeOD-d4): δ 179.9, 158.4, 158.2, 146.0, 145.6, 142.2, 142.1, 132.8, 129.3, 129.3, 123.3, 123.3, 121.3, 106.8, 106.8, 95.1, 95.0, 79.7, 66.9, 57.3, 48.4, 41.2, 33.4, 30.6, 28.9, 24.2, 0.2

### (7-2) Production of 2,7-Di(Trimethylsilylethynyl)-Fmoc-Arg(Pbf)-OH

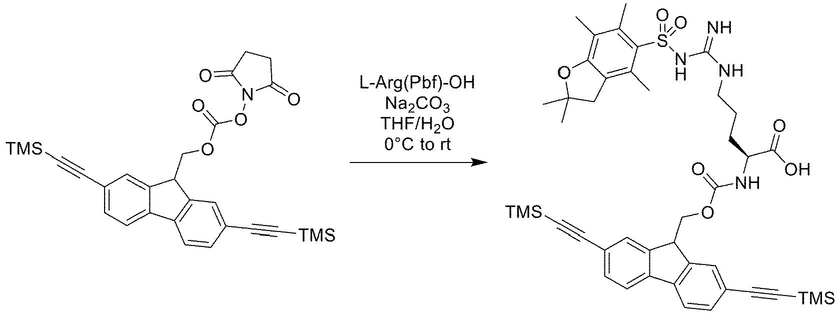

A tetrahydrofuran (THF) (6.4 mL) solution of 2,7-di(trimethylsilylethynyl)-Fmoc-N-hydroxysuccinimide ester (500 mg, 0.944 mmol) was added at 0°C to a water (4.0 mL)-tetrahydrofuran (THF) (2.0 mL) solution of H-Arg(Pbf)-OH (444 mg, 1.04 mmol) and Na₂CO₃ (150 mg, 1.42 mmol), followed by stirring at room temperature for 30 minutes.

Then, diethyl ether and water were added, 1N HCl was added to adjust the pH of the reaction liquid to 2, and the organic layer was extracted with diethyl ether (×2), dried over magnesium sulfate (MgSO₄), and then concentrated.

Then, hexane was added to the obtained residue for crystallization, followed by filtration (washing with hexane), thereby obtaining 2,7-di(trimethylsilylethynyl)-Fmoc-Arg(Pbf)-OH (640 mg, 0.761 mmol, 810) as a yellow solid.

### NMR

¹H-NMR (MeOD-d4): δ 7.75-7.71 (m, 4H), 7.47-7.44 (m, 2H), 4.39 (d, 2H, J = 6.3 Hz), 4.21 (t, 1H, J = 6.3 Hz), 4.11-4.07 (m, 1H), 3.19-3.15 (m, 2H), 2.96 (s, 2H), 2.57 (s, 3H), 2.50 (s, 3H), 2.06 (s, 3H), 1.89-1.78 (m, 1H), 1.69-1.60 (m, 1H), 1.57-1.51 (m, 2H), 1.42 (s, 6H), 0.24 (s, 18H)
¹³C-NMR (MeOD-d4): δ 175.6, 159.8, 158.5, 158.1, 146.0, 145.7, 142.3, 142.2, 139.4, 134.3, 133.5, 132.9, 129.5, 126.0, 123.4, 123.4, 121.4, 118.4, 106.8, 95.1, 87.6, 67.1, 55.0, 48.4, 43.9, 41.5, 29.9, 28.7, 19.6, 18.4, 12.5, 0.1

### (7-3) Production of 2,7-Di(Trimethylsilylethynyl)-Fmoc-His(Trt)-OH

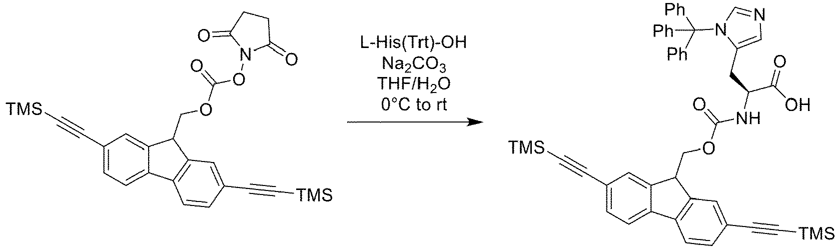

A tetrahydrofuran (THF) (6.4 mL) solution of 2,7-di(trimethylsilylethynyl)-Fmoc-N-hydroxysuccinimide ester (500 mg, 0.944 mmol) was added at 0°C to a water (4.0 mL)-tetrahydrofuran (THF) (2.0 mL) solution of H-His(Trt)-OH (644 mg, 1.04 mmol) and Na₂CO₃ (150 mg, 1.42 mmol), followed by stirring at room temperature for 30 minutes.

Then, diethyl ether and water were added, 1N HCl was added to adjust the pH of the reaction liquid to 2, and the organic layer was extracted with diethyl ether (×2), dried over magnesium sulfate (MgSO₄), and then concentrated.

Then, hexane was added to the obtained residue for crystallization, followed by filtration (washing with hexane), thereby obtaining 2,7-di(trimethylsilylethynyl)-Fmoc-His(Trt)-OH (545 mg, 0.671 mmol, 710) as a yellow solid.

### NMR

¹H-NMR (MeOD-d4): δ 7.72-7.63 (m, 5H), 7.47-7.44 (m, 2H), 7.30-7.27 (m, 9H), 7.07-7.03 (m, 6H), 6.83 (s, 1H), 4.42 (dd, 1H, J = 4.5 Hz, 8.3 Hz), 4.32 (d, 2H, J = 6.9 Hz), 4.05 (t, 1H, J = 6.9 Hz), 3.15 (dd, 1H, J = 14.7 Hz, 4.5 Hz), 2.91 (dd, 1H, J = 14.7 Hz, 8.3 Hz), 0.21 (s, 18H)
¹³C-NMR (MeOD-d4): δ 175.3, 158.1, 145.8, 145.8, 143.1, 142.2, 139.0, 136.8, 133.0, 130.9, 130.8, 129.5, 129.4, 129.3, 123.4, 121.4, 121.4, 106.8, 95.2, 77.5, 67.4, 55.9, 48.3, 31.3, 0.1, 0.1

### (7-4) Production of 2,7-Di(Trimethylsilylethynyl)-Fmoc-Phe-OH

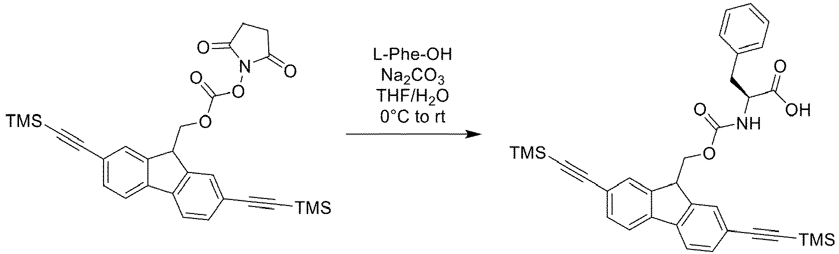

A tetrahydrofuran (THF) (6.0 mL) solution of 2,7-di(trimethylsilylethynyl)-Fmoc-N-hydroxysuccinimide ester (500 mg, 0.944 mmol) was added at 0°C to a water (1.0 mL)-tetrahydrofuran (THF) (2.4 mL) solution of H-Phe-OH (164 mg, 0.991 mmol) and Na₂CO₃ (150 mg, 1.42 mmol), followed by stirring at room temperature for 30 minutes.

Then, diethyl ether and water were added, 1N HCl was added to adjust the pH of the reaction liquid to 2, and the organic layer was extracted with diethyl ether (×2), dried over magnesium sulfate (MgSO₄), and then concentrated.

Then, hexane was added to the obtained residue for crystallization, followed by filtration (washing with hexane), thereby obtaining 2,7-di(trimethylsilylethynyl)-Fmoc-Phe-OH (385 mg, 0.664 mmol, 70%) as a yellow solid.

### NMR

¹H-NMR (MeOD-d4): δ 7.76-7.66 (m, 4H), 7.50-7.46 (m, 2H), 7.30-7.13 (m, 5H), 4.46-4.38 (m, 1H), 4.36-4.31 (m, 1H), 4.20-4.11 (m, 2H), 3.20 (dd, 1H, J = 4.8 Hz, 13.8 Hz), 2.93 (dd, 1H, J = 9.6 Hz, 18.4 Hz), 0.26 (s, 9H), 0.24 (s, 9H)
¹³C-NMR (MeOD-d4): δ 175.0, 158.2, 145.8, 145.8, 142.2, 138.7, 132.9, 130.4, 129.6, 129.5, 127.7, 123.5, 123.4, 121.3, 106.8, 106.8, 95.0, 67.3, 56.9, 48.2, 38.7, 0.1, 0.0

### (7-5) Production of 2,7-Di(Trimethylsilylethynyl)-Fmoc-Val-OH

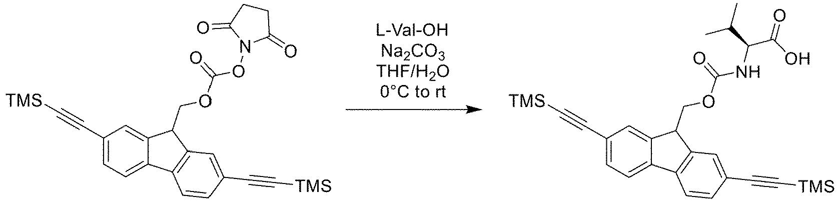

A tetrahydrofuran (THF) (7.0 mL) solution of 2,7-di(trimethylsilylethynyl)-Fmoc-N-hydroxysuccinimide ester (600 mg, 1.13 mmol) was added at 0°C to a water (2.3 mL)-tetrahydrofuran (THF) (2.2 mL) solution of H-Val-OH (164 mg, 1.40 mmol) and Na₂CO₃ (180 mg, 1.70 mmol), followed by stirring at room temperature for 1 hour.

Then, diethyl ether and water were added, 1N HCl was added to adjust the pH of the reaction liquid to 2, and the organic layer was extracted with diethyl ether (×2), dried over magnesium sulfate (MgSO₄), and then concentrated.

Then, hexane was added to the obtained residue for crystallization, followed by filtration (washing with hexane), thereby obtaining 2,7-di(trimethylsilylethynyl)-Fmoc-Val-OH (431 mg, 0.810 mmol, 72%) as a yellow solid.

### NMR

¹H-NMR (MeOD-d4): δ 7.76-7.72 (m, 4H), 7.48-7.45 (m, 2H), 4.44-4.22 (m, 3H), 4.08 (m, 1H), 2.17 (m, 1H), 0.98 (m, 6H), 0.25 (s, 18H)
¹³C-NMR (MeOD-d4): δ 175.3, 158.7, 146.1, 145.5, 142.3, 142.2, 132.9, 129.6, 129.5, 123.5, 123.5, 121.4, 106.8, 106.7, 95.0, 94.9, 67.2, 60.9, 48.3, 31.7, 19.8, 18.3, 0.0

### (7-6) Production of 2,7-Di(Trimethylsilylethynyl)-Fmoc-Met-OH

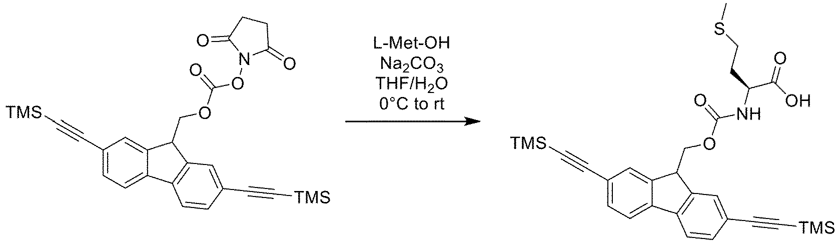

A tetrahydrofuran (THF) (7.0 mL) solution of 2,7-di(trimethylsilylethynyl)-Fmoc-N-hydroxysuccinimide ester (600 mg, 1.13 mmol) was added at 0°C to a water (4.6 mL)-tetrahydrofuran (THF) (2.2 mL) solution of H-Met-OH (185 mg, 1.24 mmol) and Na₂CO₃ (180 mg, 1.70 mmol), followed by stirring at room temperature for 30 minutes.

Then, diethyl ether and water were added, 1N HCl was added to adjust the pH of the reaction liquid to 2, and the organic layer was extracted with diethyl ether (×2), dried over magnesium sulfate (MgSO₄), and then concentrated.

Then, hexane was added to the obtained residue for crystallization, followed by filtration (washing with hexane), thereby obtaining 2,7-di(trimethylsilylethynyl)-Fmoc-Met-OH (459 mg, 0.814 mmol, 72%) as a yellow solid.

### NMR

¹H-NMR (MeOD-d4): δ 7.75-7.71 (m, 4H), 7.48-7.45 (m, 2H), 4.44-4.31 (m, 3H), 4.25-4.21 (m, 1H), 2.63-2.46 (m, 2H), 2.18-2.05 (m, 1H), 2.08 (s, 3H), 2.01-1.89 (m, 1H), 0.25 (s, 18H)

### NMR

¹³C-NMR (MeOD-d4): δ 175.6, 158.6, 146.0, 145.6, 142.3, 142.2, 132.9, 129.5, 129.5, 123.4, 123.4, 121.3, 106.8, 106.7, 95.0, 95.0, 67.2, 54.2, 48.3, 32.1, 31.4, 15.3, 0.1

### (7-7) Production of 2,7-Di(Trimethylsilylethynyl)-Fmoc-Trp-OH

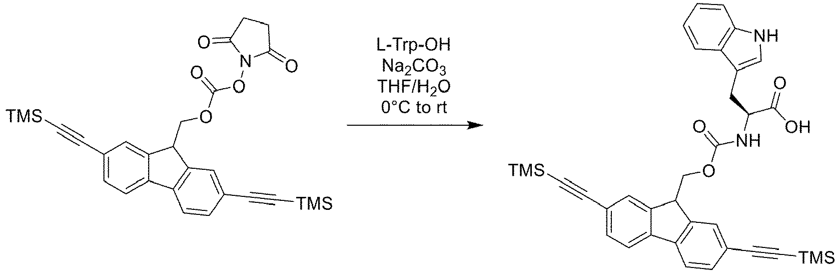

A tetrahydrofuran (THF) (7.0 mL) solution of 2,7-di(trimethylsilylethynyl)-Fmoc-N-hydroxysuccinimide ester (600 mg, 1.13 mmol) was added at 0°C to a water (4.6 mL) - tetrahydrofuran (THF) (2.2 mL) solution of H-Trp-OH (253 mg, 1.24 mmol) and Na₂CO₃ (180 mg, 1.70 mmol), followed by stirring at room temperature for 30 minutes.

Then, diethyl ether and water were added, 1N HCl was added to adjust the pH of the reaction liquid to 2, and the organic layer was extracted with diethyl ether (×2), dried over magnesium sulfate (MgSO₄), and then concentrated.

Then, hexane was added to the obtained residue for crystallization, followed by filtration (washing with hexane), thereby obtaining 2,7-di(trimethylsilylethynyl)-Fmoc-Trp-OH (586 mg, 0.947 mmol, 84%) as a yellow solid.

### NMR

¹H-NMR (MeOD-d4): δ 7.75-7.69 (m, 4H), 7.60-7.58 (m, 1H), 7.48-7.45 (m, 2H), 7.33-7.29 (m, 1H), 7.09-6.98 (m, 3H), 4.52-4.48 (m, 1H), 4.34-4.20 (m, 2H), 4.15-4.11 (m, 1H), 3.38-3.31 (m, 1H), 3.19-3.12 (m, 1H), 0.25 (s, 9H), 0.23 (s, 9H)
¹³C-NMR (MeOD-d4): δ 175.6, 158.3, 145.9, 145.8, 142.2, 138.0, 132.9, 129.5, 129.5, 128.8, 124.5, 123.4, 122.4, 121.3, 119.9, 119.3, 112.2, 111.2, 106.8, 95.0, 67.3, 56.5, 48.3, 28.8, 0.0, 0.0

### (7-8) Production of 2,7-Di(Trimethylsilylethynyl)-Fmoc-Pro-OH

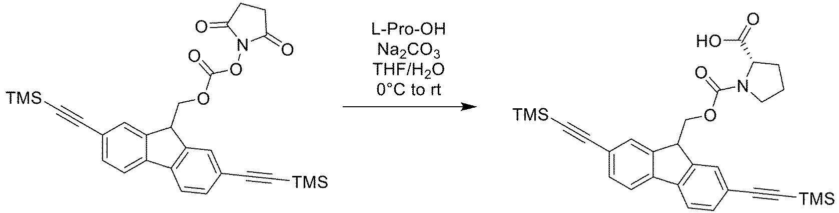

A tetrahydrofuran (THF) (7.0 mL) solution of 2,7-di(trimethylsilylethynyl)-Fmoc-N-hydroxysuccinimide ester (600 mg, 1.13 mmol) was added at 0°C to a water (4.6 mL)-tetrahydrofuran (THF) (2.2 mL) solution of H-Pro-OH (143 mg, 1.24 mmol) and Na₂CO₃ (180 mg, 1.70 mmol), followed by stirring at room temperature for 30 minutes.

Then, diethyl ether and water were added, 1N HCl was added to adjust the pH of the reaction liquid to 2, and the organic layer was extracted with diethyl ether (×2), dried over magnesium sulfate (MgSO₄), and then concentrated.

Then, hexane was added to the obtained residue for crystallization, followed by filtration (washing with hexane), thereby obtaining 2,7-di(trimethylsilylethynyl)-Fmoc-Pro-OH (477 mg, 0.900 mmol, 80%) as a yellow solid.

### NMR

¹H-NMR (MeOD-d4): δ 7.77-7.66 (m, 4H), 7.48-7.45 (m, 2H), 4.49-4.24 (m, 3H), 4.19-4.10 (m, 1H), 3.54-3.33 (m, 2H), 2.33-2.18 (m, 1H), 2.06-1.84 (m, 3H), 0.25 (s, 18H)
¹³C-NMR (MeOD-d4): δ 176.1, 175.9, 156.5, 156.3, 146.1, 146.1, 145.7, 145.6, 142.5, 142.4, 142.3, 142.3, 132.9, 132.9, 132.9, 132.8, 129.8, 129.6, 129.6, 129.4, 123.5, 123.5, 121.5, 106.9, 106.9, 106.8, 95.2, 95.1, 68.0, 67.8, 60.6, 60.1, 48.5, 48.1, 48.1, 47.7, 32.0, 31.1, 25.4, 24.4, 0.1

### (7-9) Production of 2,7-Di(Trimethylsilylethynyl)-Fmoc-Leu-OH

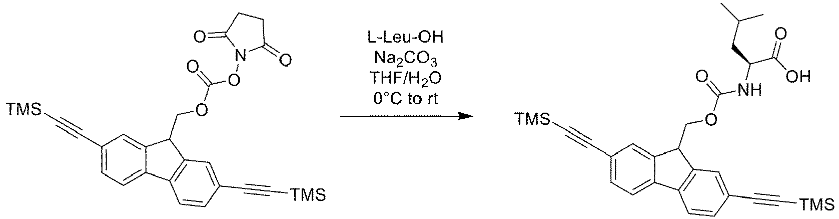

A tetrahydrofuran (THF) (7.0 mL) solution of 2,7-di(trimethylsilylethynyl)-Fmoc-N-hydroxysuccinimide ester (600 mg, 1.13 mmol) was added at 0°C to a water (4.6 mL)-tetrahydrofuran (THF) (2.2 mL) solution of H-Leu-OH (163 mg, 1.24 mmol) and Na₂CO₃ (180 mg, 1.70 mmol), followed by stirring at room temperature for 1 hour.

Then, diethyl ether and water were added, 1N HCl was added to adjust the pH of the reaction liquid to 2, and the organic layer was extracted with diethyl ether (×2), dried over magnesium sulfate (MgSO₄), and then concentrated.

Then, hexane was added to the obtained residue for crystallization, followed by filtration (washing with hexane), thereby obtaining 2,7-di(trimethylsilylethynyl)-Fmoc-Leu-OH (548 mg, 1.00 mmol, 89%) as a yellow solid.

### NMR

¹H-NMR (MeOD-d4): δ 7.76-7.71 (m, 4H), 7.49-7.46 (m, 2H), 4.44 (dd, 1H, J = 6.9 Hz, 10.5 Hz), 4.34 (dd, 1H, J = 6.0 Hz, 10.5 Hz), 4.26-4.16 (m, 2H), 1.72-1.57 (m, 2H), 1.35-1.31 (m, 1H), 0.97-0.92 (m, 6H), 0.25 (s, 18H)
¹³C-NMR (MeOD-d4): δ 176.5, 158.6, 146.1, 145.6, 142.3, 142.2, 132.9, 129.5, 123.4, 121.3, 106.8, 106.8, 95.0, 94.9, 67.1, 53.8, 48.4, 41.6, 26.0, 23.5, 21.8, 0.0

### (7-10) Production of 2,7-Di(Trimethylsilylethynyl)-Fmoc-Ile-OH

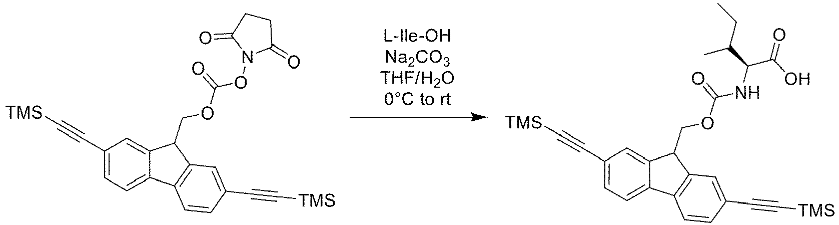

A tetrahydrofuran (THF) (7.0 mL) solution of 2,7-di(trimethylsilylethynyl)-Fmoc-N-hydroxysuccinimide ester (600 mg, 1.13 mmol) was added at 0°C to a water (4.6 mL) - tetrahydrofuran (THF) (2.2 mL) solution of H-Ile-OH (163 mg, 1.24 mmol) and Na₂CO₃ (180 mg, 1.70 mmol), followed by stirring at room temperature for 5 minutes.

Then, diethyl ether and water were added, 1N HCl was added to adjust the pH of the reaction liquid to 2, and the organic layer was extracted with diethyl ether (×2), dried over magnesium sulfate (MgSO₄), and then concentrated.

Then, hexane was added to the obtained residue for crystallization, followed by filtration (washing with hexane), thereby obtaining 2,7-di(trimethylsilylethynyl)-Fmoc-Ile-OH (518 mg, 0.948 mmol, 84%) as a yellow solid.

### NMR

¹H-NMR (MeOD-d4): δ 7.77-7.71 (m, 4H), 7.48-7.45 (m, 2H), 4.43 (dd, 1H, J = 6.9 Hz, 10.5 Hz), 4.32 (dd, 1H, J = 6.3 Hz, 10.5 Hz), 4.24 (dd, 1H, J = 6.9 Hz, 6.3 Hz), 1.95-1.82 (m, 1H), 1.56-1.43 (m, 1H), 1.32-1.18 (m, 1H), 0.97-0.88 (m, 6H), 0.25 (s, 18H)
¹³C-NMR (MeOD-d4): δ 175.3, 158.6, 146.1, 145.5, 142.3, 142.2, 132.9, 129.5, 123.5, 123.5, 121.3, 106.8, 106.8, 95.0, 94.9, 67.2, 60.1, 48.3, 38.3, 26.2, 16.2, 11.9, 0.0

### (7-11) Production of 2,7-Di(Trimethylsilylethynyl)-Fmoc-Ala-OH

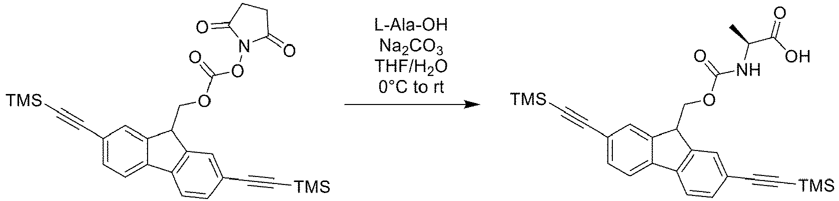

A tetrahydrofuran (THF) (6.0 mL) solution of 2,7-di(trimethylsilylethynyl)-Fmoc-N-hydroxysuccinimide ester (495 mg, 0.934 mmol) was added at 0°C to a water (4.0 mL) - tetrahydrofuran (THF) (2.4 mL) solution of H-Ala-OH (92 mg, 1.03 mmol) and Na₂CO₃ (148 mg, 1.40 mmol), followed by stirring at room temperature for 15 minutes. Further, H-Ala-OH (46 mg, 0.52 mmol) was added to the reaction liquid, followed by stirring at room temperature for 20 minutes.

Then, diethyl ether and water were added, 1N HCl was added to adjust the pH of the reaction liquid to 2, and the organic layer was extracted with diethyl ether (×2), dried over magnesium sulfate (MgSO₄), and then concentrated.

Then, hexane was added to the obtained residue for crystallization, followed by filtration (washing with hexane), thereby obtaining 2,7-di(trimethylsilylethynyl)-Fmoc-Ala-OH (369 mg, 0.733 mmol, 78%) as a yellow solid.

### NMR

¹H-NMR (MeOD-d4): δ 7.78-7.72 (m, 4H), 7.49-7.46 (m, 2H), 4.37 (d, 1H, J = 6.6 Hz), 4.36 (d, 1H, J = 6.6 Hz), 4.24 (t, 1H, J = 6.6 Hz), 4.18 (q, 1H, J = 7.2 Hz), 1.40 (d, 3H, J = 7.2 Hz), 0.25 (s, 18H)
¹³C-NMR (MeOD-d4): δ 176.5, 158.3, 146.0, 145.6, 142.2, 132.9, 129.4, 123.4, 121.3, 106.7, 95.0, 67.3, 50.9, 48.3, 17.9, 0.0

### (7-12) Production of 2,7-Di(Trimethylsilylethynyl)-Fmoc-Gly-OH

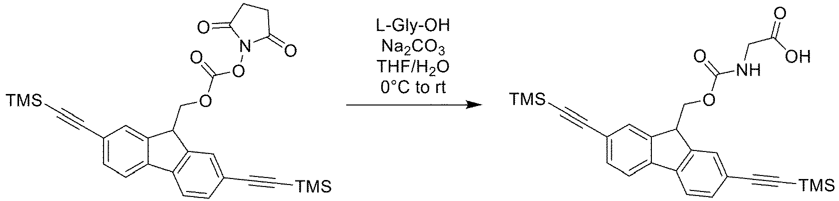

A tetrahydrofuran (THF) (6.0 mL) solution of 2,7-di(trimethylsilylethynyl)-Fmoc-N-hydroxysuccinimide ester (495 mg, 0.934 mmol) was added at 0°C to a water (4.0 mL) - tetrahydrofuran (THF) (2.4 mL) solution of H-Gly-OH (77 mg, 1.03 mmol) and Na₂CO₃ (148 mg, 1.40 mmol), followed by stirring at room temperature for 25 minutes. Further, H-Gly-OH (43 mg, 0.57 mmol) was added to the reaction liquid, followed by stirring at room temperature for 30 minutes.

Then, diethyl ether and water were added, 1N HCl was added to adjust the pH of the reaction liquid to 2, and the organic layer was extracted with diethyl ether (×2), dried over magnesium sulfate (MgSO₄), and then concentrated.

Then, hexane was added to the obtained residue for crystallization, followed by filtration (washing with hexane), thereby obtaining 2,7-di(trimethylsilylethynyl)-Fmoc-Gly-OH (378 mg, 0.772 mmol, 83%) as a yellow solid.

### NMR

¹H-NMR (MeOD-d4): δ 7.77-7.73 (m, 4H), 7.49-7.46 (m, 2H), 4.36 (d, 2H, J = 6.6 Hz), 4.24 (t, 1H, J = 6.6 Hz), 3.84 (s, 2H), 0.25 (s, 18H)
¹³C-NMR (MeOD-d4): δ 173.6, 159.0, 145.8, 142.2, 132.9, 129.5, 123.5, 121.3, 106.8, 95.0, 67.5, 48.3, 43.1, 0.0

### (7-13) Production of 2,7-Di(Trimethylsilylethynyl)-Fmoc-Ser(tBu)-OH

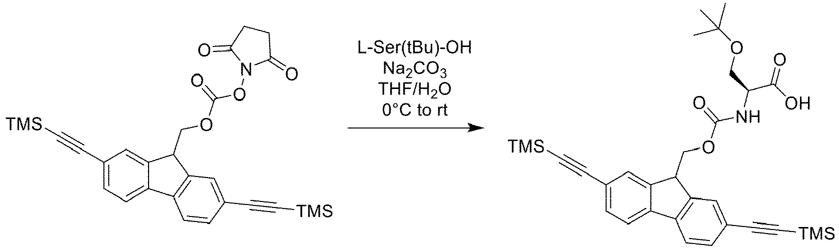

A tetrahydrofuran (THF) (6.0 mL) solution of 2,7-di(trimethylsilylethynyl)-Fmoc-N-hydroxysuccinimide ester (600 mg, 1.13 mmol) was added at 0°C to a water (4.0 mL)-tetrahydrofuran (THF) (2.4 mL) solution of H-Ser(tBu)-OH (200 mg, 1.24 mmol) and Na₂CO₃ (180 mg, 1.70 mmol), followed by stirring at room temperature for 30 minutes.

Then, diethyl ether and water were added, 1N HCl was added to adjust the pH of the reaction liquid to 2, and the organic layer was extracted with diethyl ether (×2), dried over magnesium sulfate (MgSO₄), and then concentrated.

Then, hexane was added to the obtained residue for crystallization, followed by filtration (washing with hexane), thereby obtaining 2,7-di(trimethylsilylethynyl)-Fmoc-Ser(tBu)-OH (475 mg, 0.825 mmol, 73%) as a yellow solid.

### NMR

¹H-NMR (MeOD-d4): δ 7.76-7.72 (m, 4H), 7.49-7.46 (m, 2H), 4.43-4.32 (m, 3H), 4.24 (t, 1H, J = 6.6 Hz), 3.78 (dd, 1H, J = 4.5, 10.5 Hz), 3.66 (dd, 1H, J = 3.6, 10.5 Hz), 1.18 (s, 9H), 0.25 (s, 18H)
¹³C-NMR (MeOD-d4): δ 173.8, 158.3, 146.0, 145.7, 142.3, 142.2, 132.9, 129.5, 129.5, 123.5, 121.4, 106.8, 95.0, 95.0, 74.6, 67.4, 63.0, 56.2, 48.3, 27.7, 0.1

### (7-14) Production of 2,7-Di(Trimethylsilylethynyl)-Fmoc-Thr(tBu)-OH

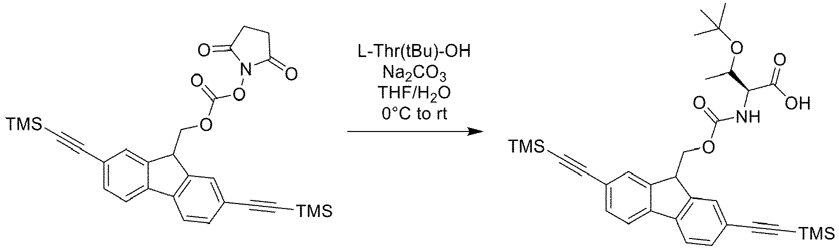

A tetrahydrofuran (THF) (6.0 mL) solution of 2,7-di(trimethylsilylethynyl)-Fmoc-N-hydroxysuccinimide ester (500 mg, 0.944 mmol) was added at 0°C to a water (4.0 mL)-tetrahydrofuran (THF) (2.4 mL) solution of H-Thr(tBu)-OH (182 mg, 1.04 mmol) and Na₂CO₃ (150 mg, 1.42 mmol), followed by stirring at room temperature for 30 minutes.

Then, diethyl ether and water were added, 1N HCl was added to adjust the pH of the reaction liquid to 2, and the organic layer was extracted with diethyl ether (×2), dried over magnesium sulfate (MgSO₄), and then concentrated.

Then, hexane was added to the obtained residue for crystallization, followed by filtration (washing with hexane), thereby obtaining 2,7-di(trimethylsilylethynyl)-Fmoc-Thr(tBu)-OH (428 mg, 0.726 mmol, 77%) as a yellow solid.

### NMR

¹H-NMR (CDCl₃): δ 7.68-7.65 (m, 4H), 7.54-7.50 (m, 2H), 5.72 (d, 1H, J = 5.1 Hz), 4.49-4.28 (m, 4H), 4.18 (t, 1H, J = 6.6 Hz), 1.34 (s, 9H), 1.14 (d, 3H, J = 6.3 Hz), 0.27 (s, 9H), 0.27 (s, 9H)
¹³C-NMR (CDCl₃): δ 171.0, 155.9, 144.3, 143.9, 141.1, 141.0, 132.2, 132.1, 128.6, 128.6, 122.3, 122.2, 120.3, 120.3, 105.5, 105.5, 95.1, 77.7, 77.4, 66.5, 58.3, 47.1, 28.1, 17.3, 0.2

### (7-15) Production of 2,7-Di(Trimethylsilylethynyl)-Fmoc-Cys(Trt)-OH

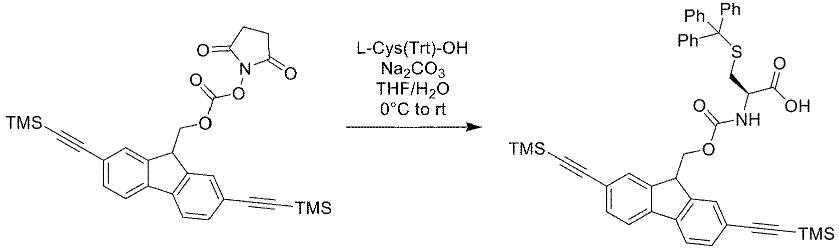

A tetrahydrofuran (THF) (6.0 mL) solution of 2,7-di(trimethylsilylethynyl)-Fmoc-N-hydroxysuccinimide ester (500 mg, 0.944 mmol) was added at 0°C to a water (4.0 mL)-tetrahydrofuran (THF) (2.4 mL) solution of H-Cys(Trt)-OH (378 mg, 1.04 mmol) and Na₂CO₃ (150 mg, 1.42 mmol), followed by stirring at room temperature for 30 minutes.

Then, diethyl ether and water were added, 1N HCl was added to adjust the pH of the reaction liquid to 2, and the organic layer was extracted with diethyl ether (×2), dried over magnesium sulfate (MgSO₄), and then concentrated.

Then, hexane was added to the obtained residue for crystallization, followed by filtration (washing with hexane), thereby obtaining 2,7-di(trimethylsilylethynyl)-Fmoc-Cys(Trt)-OH (324 mg, 0.417 mmol, 44%) as a yellow solid.

### NMR

¹H-NMR (MeOD-d4): δ 7.75-7.70 (m, 4H), 7.49-7.37 (m, 8H), 7.30-7.17 (m, 9H), 4.43-4.38 (m, 1H), 4.30-4.19 (m, 2H), 3.95 (dd, 1H, J = 9.5 Hz, 4.8 Hz), 2.67 (dd, 1H, J = 12.9 Hz, 9.5 Hz), 2.59 (dd, 1H, J = 12.9 Hz, 4.8 Hz), 0.23 (s, 18H)
¹³C-NMR (MeOD-d4): δ 173.8, 158.1, 146.0, 145.8, 142.3, 142.2, 132.9, 132.9, 130.8, 129.0, 127.9, 123.5, 121.3, 121.3, 106.8, 95.0, 68.2, 67.4, 54.8, 48.3, 34.7, 0.1

### (7-16) Production of 2,7-Di(Trimethylsilylethynyl)-Fmoc-Tyr(tBu)-OH

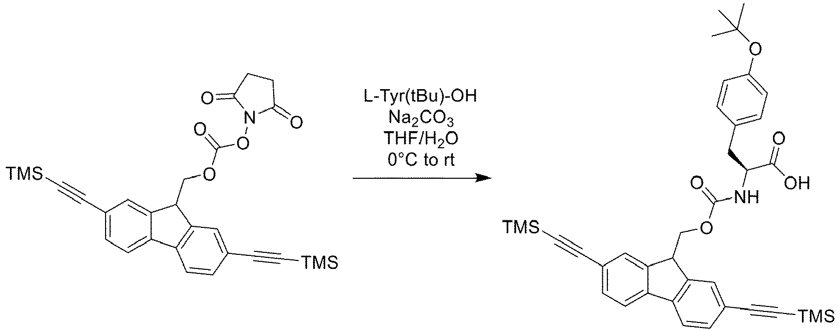

A tetrahydrofuran (THF) (6.0 mL) solution of 2,7-di(trimethylsilylethynyl)-Fmoc-N-hydroxysuccinimide ester (500 mg, 0.944 mmol) was added at 0°C to a water (4.0 mL)-tetrahydrofuran (THF) (2.4 mL) solution of H-Tyr(tBu)-OH (246 mg, 1.04 mmol) and Na₂CO₃ (150 mg, 1.42 mmol), followed by stirring at room temperature for 30 minutes.

Then, diethyl ether and water were added, 1N HCl was added to adjust the pH of the reaction liquid to 2, and the organic layer was extracted with diethyl ether (×2), dried over magnesium sulfate (MgSO₄), and then concentrated.

Then, hexane was added to the obtained residue for crystallization, followed by filtration (washing with hexane), thereby obtaining 2,7-di(trimethylsilylethynyl)-Fmoc-Tyr(tBu)-OH (466 mg, 0.715 mmol, 76%) as a yellow solid.

### NMR

¹H-NMR (MeOD-d4): δ 7.76-7.67 (m, 4H), 7.49-7.46 (m, 2H), 7.18 (d, 2H, J = 8.4 Hz), 6.89 (d, 2H, J = 8.4 Hz), 4.44-4.39 (m, 1H), 4.32-4.25 (m, 1H), 4.14-4.07 (m, 2H), 3.23-3.17 (m, 1H), 2.90-2.82 (m, 1H), 1.17 (s, 9H), 0.26 (s, 9H), 0.24 (s, 9H)
¹³C-NMR (MeOD-d4): δ 175.0, 158.1, 155.2, 145.8, 145.7, 142.2, 133.9, 132.9, 131.0, 129.7, 129.6, 125.2, 123.5, 123.4, 121.3, 106.8, 95.1, 79.4, 67.4, 57.0, 48.1, 38.5, 29.1, -0.1

### (7-17) Production of 2,7-Di(Trimethylsilylethynyl)-Fmoc-Asn(Trt)-OH

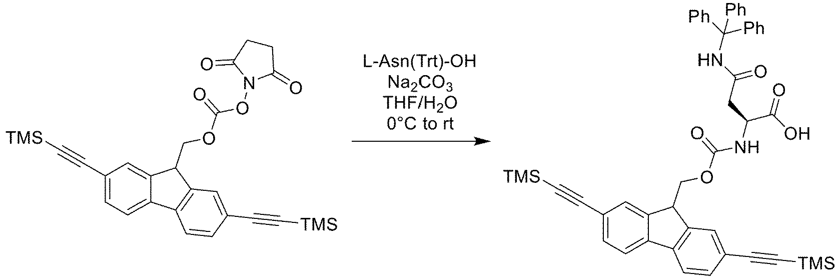

A tetrahydrofuran (THF) (6.0 mL) solution of 2,7-di(trimethylsilylethynyl)-Fmoc-N-hydroxysuccinimide ester (500 mg, 0.944 mmol) was added at 0°C to a water (4.0 mL)-tetrahydrofuran (THF) (2.4 mL) solution of H-Asn(Trt)-OH (389 mg, 1.04 mmol) and Na₂CO₃ (150 mg, 1.42 mmol), followed by stirring at room temperature for 30 minutes.

Then, diethyl ether and water were added, 1N HCl was added to adjust the pH of the reaction liquid to 2, and the organic layer was extracted with diethyl ether (×2), dried over magnesium sulfate (MgSO₄), and then concentrated.

Then, hexane was added to the obtained residue for crystallization, followed by filtration (washing with hexane), thereby obtaining 2,7-di(trimethylsilylethynyl)-Fmoc-Asn(Trt)-OH (609 mg, 0.793 mmol, 84%) as a yellow solid.

### NMR

¹H-NMR (MeOD-d4): δ 7.75-7.72 (m, 4H), 7.48-7.46 (m, 2H), 7.26-7.15 (m, 15H), 4.49-4.38 (m, 3H), 4.17 (t, 1H, J = 5.7 Hz), 2.83-2.80 (m, 2H), 0.24 (s, 9H), 0.23 (s, 9H)
¹³C-NMR (MeOD-d4): δ 174.7, 171.6, 158.3, 145.9, 145.8, 145.8, 142.3, 133.0, 132.9, 130.0, 129.3, 129.3, 128.7, 127.8, 123.5, 123.5, 121.3, 106.8, 95.1, 95.1, 71.8, 67.3, 52.5, 48.4, 39.5, 0.1

### (7-18) Production of 2,7-Di(Trimethylsilylethynyl)-Fmoc-Gln(Trt)-OH

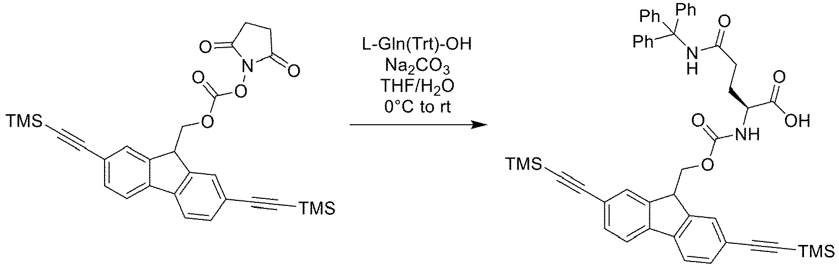

A tetrahydrofuran (THF) (6.0 mL) solution of 2,7-di(trimethylsilylethynyl)-Fmoc-N-hydroxysuccinimide ester (500 mg, 0.944 mmol) was added at 0°C to a water (4.0 mL)-tetrahydrofuran (THF) (2.4 mL) solution of H-Gln(Trt)-OH (404 mg, 1.04 mmol) and Na₂CO₃ (150 mg, 1.42 mmol), followed by stirring at room temperature for 30 minutes.

Then, diethyl ether and water were added, 1N HCl was added to adjust the pH of the reaction liquid to 2, and the organic layer was extracted with diethyl ether (×2), dried over magnesium sulfate (MgSO₄), and then concentrated.

Then, hexane was added to the obtained residue for crystallization, followed by filtration (washing with hexane), thereby obtaining 2,7-di(trimethylsilylethynyl)-Fmoc-Gln(Trt)-OH (630 mg, 0.820 mmol, 87%) as a yellow solid.

### NMR

¹H-NMR (MeOD-d4): δ 7.74-7.72 (m, 4H), 7.47-7.44 (m, 2H), 7.28-7.15 (m, 15H), 4.36 (d, 2H, J = 6.9 Hz), 4.22-4.15 (m, 2H), 2.52-2.34 (m, 2H), 2.20-2.08 (m, 1H), 1.91-1.78 (m, 1H), 0.24 (s, 9H), 0.24 (s, 9H)
¹³C-NMR (MeOD-d4): δ 175.4, 174.1, 158.6, 146.0, 145.9, 145.7, 142.2, 142.2, 132.9, 130.0, 129.5, 129.4, 128.7, 127.8, 123.5, 123.4, 121.4, 106.8, 106.8, 95.1, 95.1, 71.6, 67.3, 54.8, 48.4, 34.2, 28.8, 0.1

### (7-19) Production of 2,7-Di(Trimethylsilylethynyl)-Fmoc-Asp(tBu)-OH

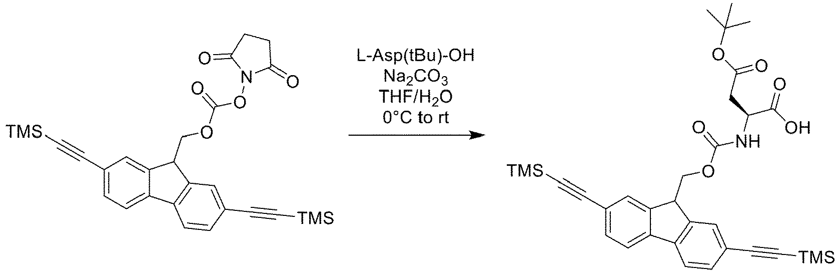

A tetrahydrofuran (THF) (7.0 mL) solution of 2,7-di(trimethylsilylethynyl)-Fmoc-N-hydroxysuccinimide ester (600 mg, 1.13 mmol) was added at 0°C to a water (4.6 mL)-tetrahydrofuran (THF) (2.2 mL) solution of H-Asp(tBu)-OH (235 mg, 1.24 mmol) and Na₂CO₃ (180 mg, 1.70 mmol), followed by stirring at room temperature for 30 minutes.

Then, diethyl ether and water were added, 1N HCl was added to adjust the pH of the reaction liquid to 2, and the organic layer was extracted with diethyl ether (×2), dried over magnesium sulfate (MgSO₄), and then concentrated.

Then, hexane was added to the obtained residue for crystallization, followed by filtration (washing with hexane), thereby obtaining 2,7-di(trimethylsilylethynyl)-Fmoc-Asp(tBu)-OH (546 mg, 0.904 mmol, 80%) as a yellow solid.

### NMR

¹H-NMR (MeOD-d4): δ 7.76-7.71 (m, 4H), 7.48-7.45 (m, 2H), 4.53 (dd, 1H, J = 5.7 Hz, 7.8 Hz), 4.44-4.33 (m, 2H), 4.23 (t, 1H, J = 6.3 Hz), 2.80 (dd, 1H, J = 5.7 Hz, 15.9 Hz), 2.65 (dd, 1H, J = 7.8 Hz, 15.9 Hz), 1.43 (s, 9H), 0.25 (s, 18H)
¹³C-NMR (MeOD-d4): δ 174.2, 171.3, 158.2, 145.9, 145.7, 142.3, 142.2, 132.9, 129.4, 129.4, 123.5, 121.4, 106.8, 95.0, 82.4, 67.4, 52.1, 48.3, 38.8, 28.4, 0.1, 0.0

### (7-20) Production of 2,7-Di(Trimethylsilylethynyl)-Fmoc-Glu(tBu)-OH

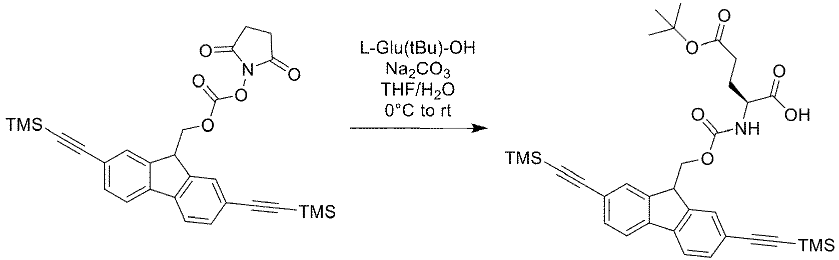

A tetrahydrofuran (THF) (7.0 mL) solution of 2,7-di(trimethylsilylethynyl)-Fmoc-N-hydroxysuccinimide ester (600 mg, 1.13 mmol) was added at 0°C to a water (4.6 mL)-tetrahydrofuran (THF) (2.2 mL) solution of H-Glu(tBu)-OH (252 mg, 1.24 mmol) and Na₂CO₃ (180 mg, 1.70 mmol), followed by stirring at room temperature for 15 minutes.

Then, diethyl ether and water were added, 1N HCl was added to adjust the pH of the reaction liquid to 2, and the organic layer was extracted with diethyl ether (×2), dried over magnesium sulfate (MgSO₄), and then concentrated.

Then, hexane was added to the obtained residue for crystallization, followed by filtration (washing with hexane), thereby obtaining 2,7-di(trimethylsilylethynyl)-Fmoc-Glu(tBu)-OH (546 mg, 0.884 mmol, 78%) as a yellow solid.

### NMR

¹H-NMR (MeOD-d4): δ 7.77-7.73 (m, 4H), 7.49-7.46 (m, 2H), 4.39-4.36 (m, 2H), 4.26-4.18 (m, 2H), 2.40-2.27 (m, 2H), 2.21-2.10 (m, 1H), 1.95-1.83 (m, 1H), 1.44 (s, 9H), 0.25 (s, 18H)
¹³C-NMR (MeOD-d4): δ 175.4, 173.8, 158.5, 146.0, 145.7, 142.3, 142.2, 132.9, 129.5, 129.5, 123.5, 123.5, 121.3, 106.8, 106.8, 95.0, 95.0, 81.8, 67.3, 54.6, 48.3, 32.8, 28.4, 28.0, 0.1

### (8) Production 5 of Amine-Protecting Group

### (8-1) Production of 2,7-Di(Phenylethynyl)-9-Fluorenylmethanol

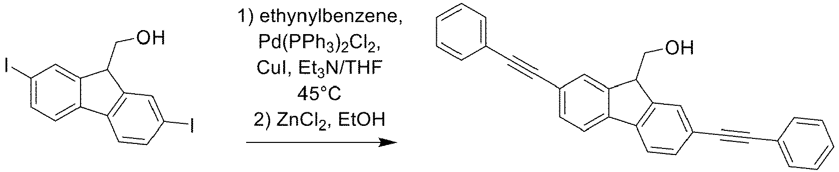

2,7-Diiodo-9-fluorenylmethanol (0.500 g, 1.16 mmol), copper(I) iodide (CuI) (22.1 mg, 0.116 mmol), and bis(triphenylphosphine)palladium(II) dichloride (Pd(PPh₃)₂Cl₂) (81.4 mg, 0.116 mmol) were dissolved in tetrahydrofuran (THF) (3.5 mL), and triethylamine (Et₃N) (0.78 mL) and ethynylbenzene (0.51 mL, 4.64 mmol) were added, followed by stirring at 45°C for 2.5 hours.

After concentration, ethanol (5.0 mL) and zinc chloride (63.3 mg, 0.464 mmol) were added, followed by stirring at room temperature overnight, and then Celite filtration (washing with ethanol) and concentration were performed.

Then, the residue was purified by silica gel column chromatography, thereby obtaining 2,7-di(phenylethynyl)-9-fluorenylmethanol (94.0 mg, 0.237 mmol, 20%) as a brown solid.

### NMR

¹H-NMR (CDCl₃): δ 7.80-7.73 (m, 4H), 7.61-7.55 (m, 6H), 7.39-7.35 (m, 6H), 4.15 (t, 1H, J = 5.7 Hz), 4.07 (d, 2H, J = 5.7 Hz)
¹³C-NMR (CDCl₃): δ 145.0, 141.1, 131.8, 131.6, 128.5, 128.4, 128.1, 123.4, 122.3, 120.4, 90.2, 90.1, 65.0, 50.2

### (8-2) Production of 2,7-Di(Phenylethynyl)-Fmoc-Chloride

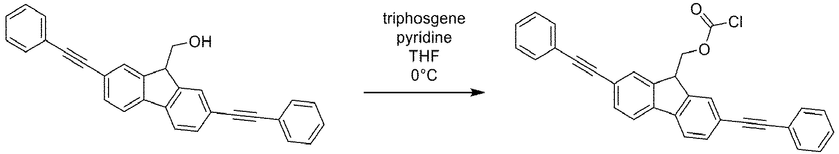

Pyridine (23.4 µL, 0.291 mmol) and a tetrahydrofuran (THF) (2.0 mL) solution of 2,7-di(phenylethynyl)-9-fluorenylmethanol (76.9 mg, 0.194 mmol) were slowly added dropwise at 0°C to a tetrahydrofuran (THF) (0.5 mL) solution of triphosgene (34.7 mg, 0.117 mmol), followed by stirring at 0°C for 20 minutes. Further, triphosgene (23 mg, 0.078 mmol) and pyridine (32 µL, 0.398 mmol) were added, followed by stirring at 0°C for 1.5 hours.

Then, water was added, followed by extraction with ethyl acetate, and the organic layer was dried over magnesium sulfide (MgS).

After concentration, 2,7-di(phenylethynyl)-Fmoc-chloride (88.0 mg, 0.192 mmol, 99%) was obtained as a brown solid.

### NMR

¹H-NMR (CDCl₃): δ 7.77-7.74 (m, 4H), 7.65-7.55 (m, 6H), 7.38-7.36 (m, 6H), 4.61 (d, 2H, J = 7.2 Hz), 4.33 (t, 1H, J = 7.2 Hz),
¹³C-NMR (CDCl₃): δ 151.0, 145.0, 1423.0, 140.8, 132.2, 131.8, 128.6, 128.3, 123.3, 122.8, 120.6, 90.6, 89.7, 72.9, 46.2

### (8-3) Production of 2,7-Di(Phenylethynyl)-Fmoc-N-Hydroxysuccinimide Ester

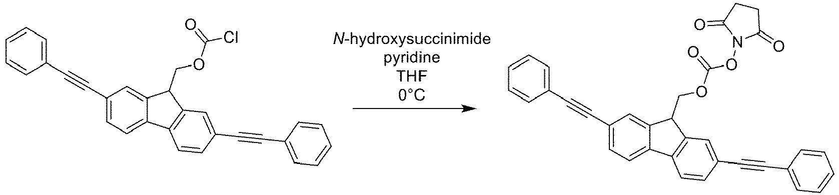

N-hydroxysuccinimide (12.2 mg, 0.106 mmol) and pyridine (7.7 µL, 0.096 mmol) were dissolved in tetrahydrofuran (THF) (0.5 mL), and a tetrahydrofuran (THF) (0.5 mL) solution of 2,7-di(phenylethynyl)-Fmoc-chloride (44 mg, 0.096 mmol) was added at 0°C to the reaction liquid, followed by stirring at the same temperature for 20 minutes.

Then, water was added to the reaction liquid, followed by extraction with ethyl acetate and drying over magnesium sulfide (MgS), and the solvent was distilled off.

Then, the residue was purified by silica gel column chromatography (hexane/ethyl acetate = 2/1 -> 5/2), thereby obtaining 2,7-di(phenylethynyl)-Fmoc-N-hydroxysuccinimide ester (46.2 mg, 0.086 mmol, 89%) as a yellow solid.

### NMR

¹H-NMR (CDCl₃): δ 7.79-7.74 (m, 4H), 7.64-7.55 (m, 6H), 7.39-7.34 (m, 6H), 4.61 (d, 2H, J = 7.5 Hz), 4.37 (t, 1H, J = 7.5 Hz), 2.86 (s, 4H)
¹³C-NMR (CDCl₃): δ 168.6, 151.7, 143.0, 140.8, 132.2, 131.8, 128.5, 128.5, 128.5, 123.4, 122.8, 120.6, 90.6, 89.8, 72.5, 46.4, 25.6

### (9) Production 3 of Special Amino Acid

### (9-1) Production of 2,7-Di(Phenylethynyl)-Fmoc-Gly-OH

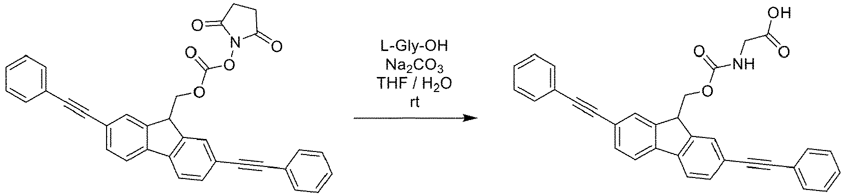

A tetrahydrofuran (THF) (1.5 mL) solution of 2,7-di(phenylethynyl)-Fmoc-N-hydroxysuccinimide ester (46 mg, 0.086 mmol) was added at room temperature to a water (1.0 mL)-tetrahydrofuran (THF) (0.5 mL) solution of H-Gly-OH (7.1 mg, 0.095 mmol) and Na₂CO₃ (13.7 mg, 0.129 mmol), followed by stirring at room temperature for 15 minutes.

Then, diethyl ether and water were added, 1N HCl was added to adjust the pH of the reaction liquid to 2, and the organic layer was extracted with diethyl ether (×2), dried over magnesium sulfide (MgS), and then concentrated.

Then, hexane was added to the obtained residue for crystallization, followed by filtration (washing with hexane), thereby obtaining 2,7-di(phenylethynyl)-Fmoc-Gly-OH (26 mg, 0.053 mmol, 62%) as a yellow solid.

### NMR

¹H-NMR (DMSO-d6): δ 12.54 (s, 1H), 8.01-7.99 (m, 2H), 7.92 (s, 2H), 7.66-7.57 (m, 7H), 7.47-7.42 (m, 6H), 4.45 (d, 2H, J = 6.3 Hz), 4.33 (t, 1H, J = 6.3 Hz), 3.65 (d, 2H, J = 3.9 Hz)
¹³C-NMR (DMSO-d6): δ 171.5, 156.4, 144.7, 141.1, 140.5, 138.9, 138.1, 132.2, 131.4, 131.3, 128.8, 128.8, 128.0, 124.5, 122.4, 122.3, 121.4, 121.2, 120.9, 120.9, 90.0, 89.9, 89.9, 59.9, 46.6, 42.1

### (10) Production 6 of Amine-Protecting Group

### (10-1) Production of 2,7-Di(Cyclopropylethynyl)-9-Fluorenylmethanol

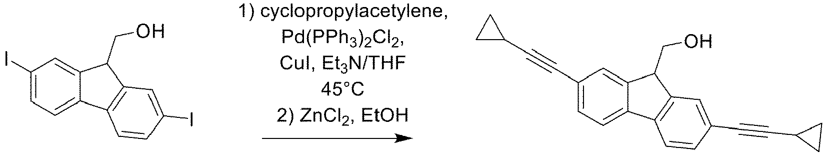

2,7-Diiodo-9-fluorenylmethanol (0.500 g, 1.16 mmol), copper(I) iodide (CuI) (22.1 mg, 0.116 mmol), and bis(triphenylphosphine)palladium(II) dichloride (Pd(PPh₃)₂Cl₂) (81.4 mg, 0.116 mmol) were dissolved in tetrahydrofuran (THF) (3.5 mL), and triethylamine (Et₃N) (0.78 mL) and cyclopropylacetylene (0.39 mL, 4.64 mmol) were added, followed by stirring at 45°C for 2 hours.

After concentration, ethanol (5.0 mL) and zinc chloride (85 mg, 0.624 mmol) were added, followed by stirring at room temperature overnight, and then Celite filtration (washing with ethanol) and concentration were performed.

Then, the residue was purified by silica gel column chromatography (hexane/ethyl acetate = 4/1 -> 3/1), thereby obtaining 2,7-di(cyclopropylethynyl)-9-fluorenylmethanol (311 mg, 0.959 mmol, 83%) as a yellow solid.

### NMR

¹H-NMR (CDCl₃): δ 7.63-7.60 (m, 4H), 7.42-7.39 (m, 2H), 4.06-3.97 (m, 3H), 1.52-1.43 (m, 2H), 0.92-0.79 (m, 8H)
¹³C-NMR (CDCl₃): δ 144.7, 140.5, 131.5, 128.1, 122.8, 120.1, 94.2, 76.4, 65.1, 50.1, 8.8, 0.4

### (10-2) Production of 2,7-Di(Cyclopropylethynyl)-Fmoc-Chloride

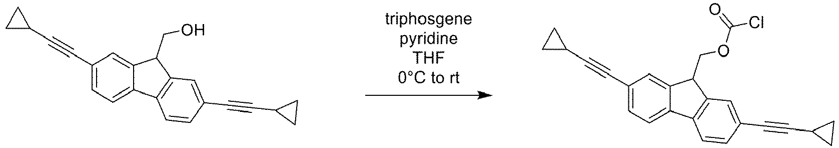

A tetrahydrofuran (THF) (1.0 mL) solution of 2,7-di(cyclopropylethynyl)-9-fluorenylmethanol (50 mg, 0.15 mmol) and pyridine (24 µL, 0.30 mmol) were added at 0°C to a tetrahydrofuran (THF) (0.5 mL) solution of triphosgene (27 mg, 0.091 mmol), followed by stirring at 0°C for 10 minutes and further stirring at room temperature for 30 minutes.

Then, water was added, followed by extraction with ethyl acetate, and the organic layer was dried over magnesium sulfide (MgS).

After concentration, 2,7-di(cyclopropylethynyl)-Fmoc-chloride (61 mg, 0.16 mmol, quant.) was obtained as a yellow solid.

### NMR

¹H-NMR (CDCl₃): δ 7.63-7.57 (m, 4H), 7.45-7.42 (m, 2H), 4.52 (d, 2H, J = 7.2 Hz), 4.21 (t, 1H, J = 7.2 Hz), 1.53-1.44 (m, 2H), 0.93-0.80 (m, 8H)
¹³C-NMR (CDCl₃): δ 150.9, 142.7, 140.2, 132.1, 128.2, 123.2, 120.3, 94.6, 76.1, 73.0, 46.1, 8.9, 0.4

### (10-3) Production of 2,7-Di(Cyclopropylethynyl)-Fmoc-N-Hydroxysuccinimide Ester

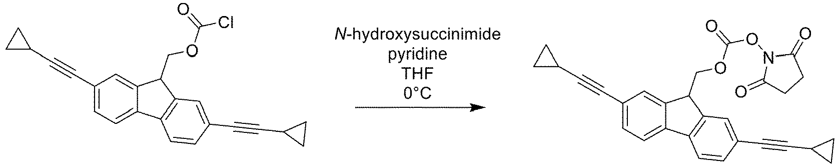

N-hydroxysuccinimide (122 mg, 1.1 mmol) and pyridine (77.3 µL, 0.20 mmol) were dissolved in tetrahydrofuran (THF) (1.0 mL), and a tetrahydrofuran (THF) (3.8 mL) solution of 2,7-di(cyclopropylethynyl)-Fmoc-chloride (370 mg, 0.96 mmol) was added at 0°C to the reaction liquid, followed by stirring at the same temperature for 20 minutes.

Then, water was added to the reaction liquid, followed by extraction with ethyl acetate and drying over magnesium sulfide (MgS), and the solvent was distilled off.

Then, hexane was added to the residue for crystallization, followed by filtration, thereby obtaining 2,7-di(cyclopropylethynyl)-Fmoc-N-hydroxysuccinimide ester (394 mg, 0.85 mmol, 88%) as a yellow solid.

### NMR

¹H-NMR (CDCl₃): δ 7.63-7.59 (m, 4H), 7.45-7.42 (m, 2H), 4.52 (d, 2H, J = 7.5 Hz), 4.24 (t, 1H, J = 7.5 Hz), 2.85 (s, 4H), 1.52-1.43 (m, 2H), 0.92-081 (m, 8H)
¹³C-NMR (CDCl₃): δ 168.6, 151.6, 142.8, 140.2, 132.1, 128.4, 123.2, 120.2, 94.6, 76.2, 72.6, 46.3, 25.6, 8.8, 0.4

### (11) Production 4 of Special Amino Acid

### (11-1) Production of 2,7-Di(Cyclopropylethynyl)-Fmoc-Gly-OH

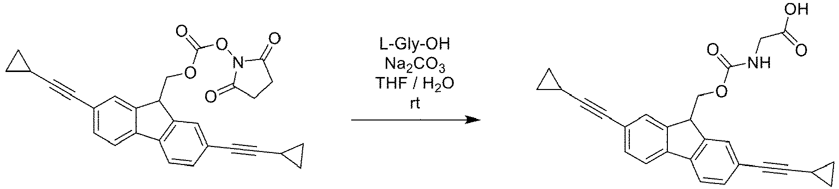

2,7-Di(cyclopropylethynyl)-Fmoc-N-hydroxysuccinimide ester (50 mg, 0.11 mmol) was added at room temperature to a water (1.0 mL)-tetrahydrofuran (THF) (2.0 mL) solution of H-Gly-OH (8.9 mg, 0.12 mmol) and Na₂CO₃ (17.1 mg, 0.16 mmol), followed by stirring at room temperature for 10 minutes.

Then, diethyl ether and water were added, 1N HCl was added to adjust the pH of the reaction liquid to 2, and the organic layer was extracted with diethyl ether (×2), dried over magnesium sulfide (MgS), and then concentrated, thereby obtaining 2,7-di(cyclopropylethynyl)-Fmoc-Gly-OH (47 mg, 0.11 mmol, quant.) as a yellow solid.

### NMR

¹H-NMR (DMSO-d6): δ 12.55 (s, 1H), 7.84-7.81 (m, 2H), 7.67 (s, 2H), 7.60 (t, 1H, J = 6.0 Hz), 7.41-7.38 (m, 2H), 4.33 (d, 2H, J = 6.3 Hz), 4.19 (t, 1H, J = 6.3 Hz), 3.64 (d, 2H, J = 6.0 Hz), 1.60-1.51 (m, 2H), 0.93-0.87 (m, 4H), 0.78-0.73 (m, 4H)
¹³C-NMR (DMSO-d6): δ 171.5, 156.4, 144.4, 139.7, 131.1, 127.9, 122.0, 120.5, 94.3, 76.2, 65.1, 46.4, 42.0, 8.5, -0.1

### (12) Production 7 of Amine-Protecting Group

### (12-1) Production of 2,7-Di(Ethynyl)-9-Fluorenylmethanol

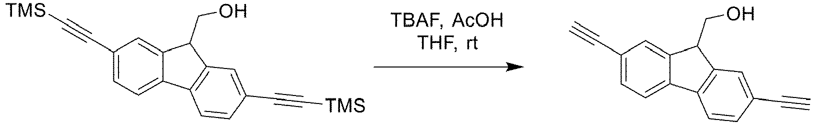

Acetic acid (AcOH, 71.5 µL, 1.25 mmol) and tetra-n-butylammonium fluoride (TBAF) (ca. 1 M in THF, 1.25 mL, 1.25 mmol) were added to a tetrahydrofuran (THF) (5.0 mL) solution of 2,7-di(trimethylsilylethynyl)-9-fluorenylmethanol (195 mg, 0.50 mmol), followed by stirring at room temperature for 10 minutes.

After concentration, water and ethyl acetate were added, the organic layer was extracted with ethyl acetate and dried over magnesium sulfide (MgS), and the solvent was distilled off.

Then, the residue was purified by silica gel column chromatography (hexane/ethyl acetate = 9/1 -> 2/1), thereby obtaining 2,7-di(ethynyl)-9-fluorenylmethanol (107 mg, 0.44 mmol, 88%) as a yellow solid.

### NMR

¹H-NMR (CDCl₃): δ 7.76-7.70 (m, 4H), 7.57-7.54 (m, 2H), 4.10 (dd, 1H, J = 5.7, 6.6 Hz), 4.02 (d, 1H, J = 6.6 Hz), 4.02 (d, 1H, J = 5.7 Hz), 3.14 (s, 2H)
¹³C-NMR (CDCl₃): δ 144.9, 141.4, 132.1, 128.7, 121.2, 120.4, 84.2, 77.9, 64.9, 50.2

### (12-2) Production of 2,7-Di(Ethynyl)-Fmoc-Chloride

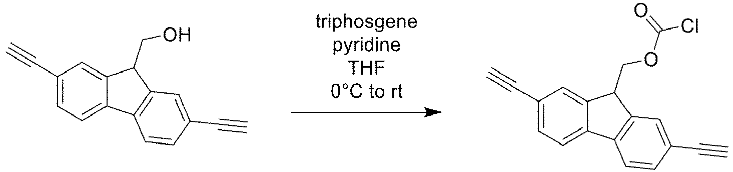

A THF (6.5 mL) solution of 2,7-di(ethynyl)-9-fluorenylmethanol (320 mg, 1.31 mmol) and pyridine (159 µL, 1.97 mmol) were added at 0°C to a tetrahydrofuran (THF) (6.5 mL) solution of triphosgene (234 mg, 0.79 mmol), followed by stirring at 0°C for 45 minutes.

Then, water was added, followed by extraction with ethyl acetate, and the organic layer was dried over magnesium sulfide (MgS).

After concentration, 2,7-di(ethynyl)-Fmoc-chloride (402 mg, 0.31 mmol, quant.) was obtained as a yellow solid.

### NMR

¹H-NMR (CDCl₃): δ 7.76-7.70 (m, 4H), 7.60-7.56 (m, 2H), 4.55 (d, 2H, J = 7.5 Hz), 4.28 (t, 1H, J = 7.5 Hz), 3.17 (s, 2H)
¹³C-NMR (CDCl₃): δ 150.9, 142.9, 141.2, 132.7, 128.9, 121.7, 120.7, 83.8, 78.3, 72.7, 46.1

### (12-3) Production of 2,7-Di(Ethynyl)-Fmoc-N-Hydroxysuccinimide Ester

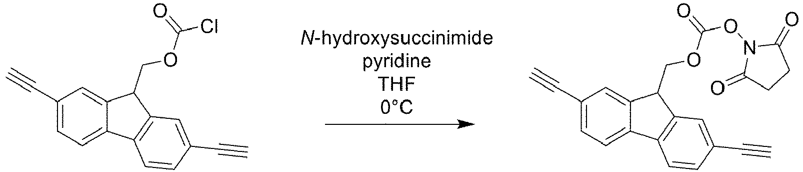

N-hydroxysuccinimide (166 mg, 1.44 mmol) and pyridine (106 µL, 1.31 mmol) were dissolved in tetrahydrofuran (THF) (6.5 mL), and a tetrahydrofuran (THF) (6.5 mL) solution of 2,7-di(ethynyl)-Fmoc-chloride (402 mg, 1.31 mmol) was added at 0°C to the reaction liquid, followed by stirring at the same temperature for 15 minutes.

Then, water was added to the reaction liquid, followed by extraction with ethyl acetate and drying over magnesium sulfide (MgS), and the solvent was distilled off.

Then, hexane was added to the residue for crystallization, followed by filtration, thereby obtaining 2,7-di(ethynyl)-Fmoc-N-hydroxysuccinimide ester (333 mg, 0.864 mmol, 66%) as a yellow solid.

### NMR

¹H-NMR (CDCl₃): δ 7.75-7.71 (m, 4H), 7.60-7.57 (m, 2H), 4.56 (d, 2H, J = 7.5 Hz), 4.33 (t, 1H, J = 7.5 Hz), 3.17 (s, 2H), 2.86 (s, 4H)
¹³C-NMR (CDCl₃): δ 168.6, 151.6, 142.9, 141.2, 132.7, 129.0, 121.7, 120.6, 83.8, 78.3, 72.3, 46.3, 26.6

### (13) Production 5 of Special Amino Acid

### (13-1) Production of 2,7-Di(Ethynyl)-Fmoc-Gly-OH

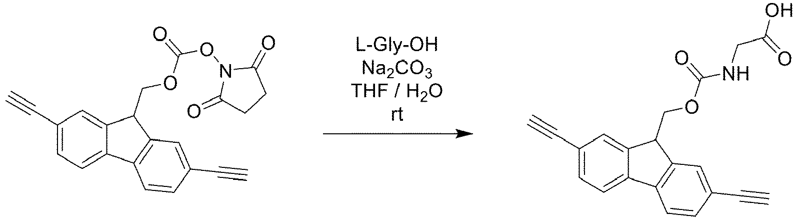

2,7-Di(ethynyl)-Fmoc-N-hydroxysuccinimide ester (100 mg, 0.26 mmol) was added at room temperature to a water (1.0 mL)-tetrahydrofuran (THF) (0.6 mL) solution of H-Gly-OH (22 mg, 0.29 mmol) and Na₂CO₃ (41 mg, 0.39 mmol), followed by stirring at the same temperature for 10 minutes.

Then, diethyl ether and water were added, 1N HCl was added to adjust the pH of the reaction liquid to 2, and the organic layer was extracted with diethyl ether (×2), dried over magnesium sulfide (MgS), and then concentrated, thereby obtaining 2,7-di(ethynyl)-Fmoc-Gly-OH (89 mg, 0.26 mmol, quant.) as a yellow solid.

### NMR

¹H-NMR (MeOD-d4): δ 7.80-7.77 (m, 4H), 7.53-7.50 (m, 2H), 4.36 (d, 2H, J = 6.6 Hz), 4.26 (t, 1H, J = 6.6 Hz), 3.84 (s, 2H), 3.53 (s, 2H)
¹³C-NMR (MeOD-d4): δ 173.6, 159.0, 145.8, 142.3, 133.0, 129.8, 122.8, 121.4, 84.8, 79.1, 67.5, 48.2, 43.1

### (13-2) Production of 2,7-Di(Bromoethynyl)-Fmoc-Gly-OH

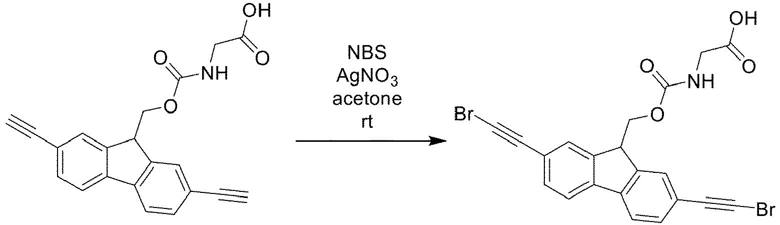

Silver nitrate (1.0 mg, 0.006 mmol) and N-bromosuccinimide (NBS) (25 mg, 0.14 mmol) were added to an acetone solution (1.0 mL) of 2,7-di(ethynyl)-Fmoc-Gly-OH (20 mg, 0.058 mmol), followed by stirring at room temperature for 4 hours.

Then, an aqueous sodium thiosulfate solution was added, the organic layer was extracted with diethyl ether and dried over magnesium sulfide (MgS), and the solvent was distilled off, thereby obtaining 2,7-di(bromoethynyl)-Fmoc-Gly-OH (24 mg, 0.048 mmol, 83%) as a yellow solid.

### NMR

¹H-NMR (MeOD-d4): δ 7.75-7.72 (m, 4H), 7.47-7.44 (m, 2H), 4.31 (d, 2H, J = 6.9 Hz), 4.20 (t, 1H, J = 6.9 Hz), 3.84 (s, 2H)
¹³C-NMR (MeOD-d4): δ 172.4, 158.9, 145.8, 142.2, 133.0, 129.7, 123.0, 121.5, 81.3, 67.4, 51.4, 48.2, 43.2

### Industrial Applicability

In the amine-protecting groups of the present invention, triple bonds are introduced into their 9-fluorenylmethyloxycarbonyl protecting groups (Fmoc), and these amine-protecting groups are novel and useful for the synthesis of special amino acids.

The amine-protecting group as the compound represented by formula (1) of the present invention is excellent in controlling the ease of deprotection.

The amine-protecting group as the compound represented by formula (1) of the present invention is excellent in controlling pKa at the α-position of Fmoc and improving the yield of special amino acids.

The amine-protecting group as the compound represented by formula (1) of the present invention is excellent in tracking various reactions based thereon because the triple bonds introduced can be detected by Raman spectroscopy.

## Claims

1. A compound represented by the following formula (1): 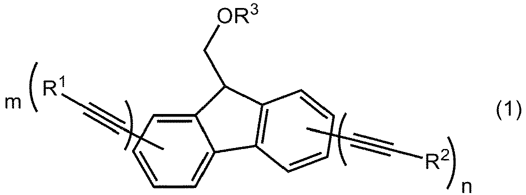 wherein
in formula (1), R¹ and R² are the same or different and are each a hydrogen atom, a halogen atom, an alkyl group, a trimethylsilyl group, an acetyl group, a benzoyl group, an ester group, an alkyl ester group, an aryl group, an alkyl alcohol group, a nitrile group, a group represented by 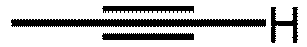 or a group represented by 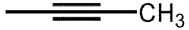
in formula (1), R³ is a hydrogen atom, a halogenated carbonyl group, or an active ester group;
in formula (1), m is an integer of 1 to 4; and
in formula (1), n is an integer of 0 to 4.

2. The compound according to claim 1, represented by the following formula (2): 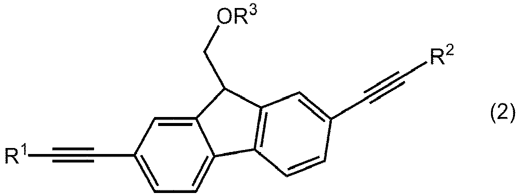 wherein
in formula (2), R¹ and R² are the same or different and are each a hydrogen atom, a halogen atom, an alkyl group, a trimethylsilyl group, an acetyl group, a benzoyl group, an ester group, an alkyl ester group, an aryl group, an alkyl alcohol group, a nitrile group, a group represented by 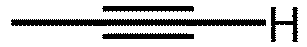 or a group represented by 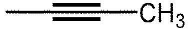
in formula (2), R³ is a hydrogen atom, a halogenated carbonyl group, or an active ester group.

3. The compound according to claim 1 or 2, wherein the active ester group is an N-hydroxysuccinimide ester group, a pentafluorophenyl ester group, a 4-nitrophenyl ester group, a 2,4-dinitrophenyl ester group, an N-hydroxyphthalimide ester group, or a 1-hydroxybenzotriazole ester group.

4. The compound according to claim 1 or 2, wherein the active ester group is an N-hydroxysuccinimide ester group or a pentafluorophenyl ester group.

5. A compound represented by the following formula (3): 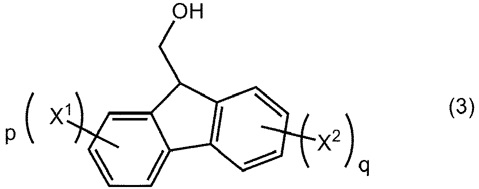 wherein
in formula (3), X¹ and X² are the same or different and are each a halogen atom;
in formula (3), p is an integer of 1 to 4; and
in formula (3), q is an integer of 0 to 4.

6. The compound according to claim 5, wherein in formula (3), X¹ and X² are each an iodine atom.
